# EUROPEAN PATENT APPLICATION

(11) **EP 1 016 731 A1**
(43) Date of publication of application: **05.07.2000**
(21) Application number: 99900152.2
(22) Date of filing: 08.01.1999
(51) Int. Cl.: C12Q 1/68

(54) **PROBES FOR DETECTING TARGET NUCLEIC ACID, METHOD OF DETECTING TARGET NUCLEIC ACID, AND SOLID PHASE FOR DETECTING TARGET NUCLEIC ACID AND PROCESS FOR PRODUCING THE SAME**

(30) Priority: 08.01.1998 JP 248298
(71) Applicant: Laboratory of Molecular Biophotonics, Hamakita-shi, Shizuoka 434-8555 (JP)
(72) Inventor: ABE, Satoshi, Laboratory of Molecular Biophotonics, Hamakita-shi, Shizuoka 434-8555 (JP); KODAMA, Hirofumi, Susono-shi, Shizuoka 410-1123 (JP)
(74) Representative: Grünecker, Kinkeldey, Stockmair & Schwanhäusser Anwaltssozietät
(86) International application number: JP9900041
(87) International publication number: WO9935287

(57) **Abstract**

This invention relates to a pair of probes for the detection of a target nucleic acid, said pair of probes comprising (1) probe 1 having base sequence B1 complementary to A1 between two specific sequential base sequences A1 and A2 of the nucleic acid, wherein (a) the 5'-terminus of base sequence B1 is phosphorylated and (b) a first solid phase immobilizing part is bound to the 3'-terminus of base sequence B1, and (2) probe 2 having base sequence B2 complementary to A2 between base sequences A1 and A2, wherein (a) the 5'-terminus of base sequence B2 is bound to one end of a cleavage part and (b) a second solid phase immobilizing part is bound to the other part of the cleavage part. When the target nucleic acid is hybridized above the solid phase of the invention where said probes are immobilized on its surface, the probes on the solid phase occupy spatial positions beneficial to the formation of a hybrid and thus forms the hybrid efficiently. Therefore, probe 1 and probe 2 are efficiently ligated by ligase reaction. Furthermore, after the target nucleic acid is removed from the hybrid, only probe 2 ligated as described above will be able to exist on the solid phase through cleavage reaction of the cleavage part. Therefore, after the free probe 2 has been removed by washing, it will become possible to detect the presence of probe 2 on the solid phase ligated as described above, with high sensitivity and high recognition. This will enable detection of the presence of a target nucleic acid with higher sensitivity and higher recognition as compared to methods in the prior art.

## Description

### TECHNICAL FIELD

This invention relates to probes for the detection of a target nucleic acid having a specific polynucleotide sequence, a solid phase for detection of the target nucleic acid, a method for preparation of the solid phase, and a method of detecting the target nucleic acid having a specific polynucleotide sequence.

### BACKGROUND ART

With the advent of research development in recent years, a variety of biological information has come to being derivable from gene sequences. Consequently, detection of the genes (which correspond to the specific polynucleotide sequences of target nucleic acids) has enabled the diagnosis of diseases, sensitivity against drugs, compatibility in organ transplantation and the like in the medical field; it has enabled the detection and identification of diverse pathogens responsible for food poisoning in the food sciences.

In order to detect such specific polynucleotide sequences (or base sequences), methods for forming hybrids (viz, hybridization techniques) have been conventionally practiced that utilize probes, which are labeled, having base sequences complementary to those to be detected. Since the base sequences to be detected vary widely in accordance with the purposes of detection, probes having a variety of base sequences depending on those purposes are employed in the detection.

However, it is pointed out that the problem common to such hybridization techniques is their poor ability to recognize the base sequences. In other words, the nucleic acid interaction by virtue of hybridization is likely to take place even if there are a few mismatches between the sequences. Therefore, the hybridization techniques are not satisfactory for a method to detect only the nucleic acid having a specific base sequence with strictness.

### DISCLOSURE OF INVENTION

This invention resides in that it solves the above-mentioned problem and provides probes for detecting a target nucleic acid conveniently and rapidly which have a high recognition ability, a solid phase for the detection of a target nucleic acid and a method for its preparation, and a method of detecting a nucleic acid.

In view of the drawback inherent in hybridization techniques in the prior art as described above, the present inventors made thorough investigations and succeeded in finding probes for the detection of a target nucleic acid, having novel structures, as well as a solid phase for the detection of a nucleic acid on which such probes are immobilized; and they further succeeded in developing a method of detecting a nucleic acid using said probes and solid phase. This invention has thus been accomplished.

Specifically, this invention provides probes for the detection of a target nucleic acid as will be described the following Items 1-8.
1. A pair of probes for the detection of a target nucleic acid comprising:
   (1) probe 1 having base sequence B1 complementary to A1 between two specific sequential base sequences A1 and A2 of the target nucleic acid, wherein (a) the 5'-terminus of base sequence B1 is phosphorylated and (b) a first solid phase immobilizing part is bound to the 3'-terminus of base sequence B1; and
   (2) probe 2 having base sequence B2 complementary to A2 between base sequences A1 and A2, wherein (a) the 5'-terminus of base sequence B2 is bound to one end of a cleavage part and (b) a second solid phase immobilizing part is bound to the other end of the cleavage part.
2. A pair of probes for the detection of a target nucleic acid comprising:
   (1) probe 1 having base sequence B1 complementary to A1 between two specific sequential base sequences A1 and A2 of the target nucleic acid, wherein (a) the 5'-terminus of base sequence B1 is phosphorylated, (b) the 3'-terminus of base sequence B1 is bound to one end of a cleavage part, and (c) a first solid phase immobilizing part is bound to the other part of the cleavage part; and
   (2) probe 2 having base sequence B2 complementary to A2 between base sequences A1 and A2, wherein a second solid phase immobilizing part is bound to the 5'-terminus of base sequence B2.
3. The probes for the detection of a target nucleic acid as described is Item 1, wherein either (a) probe 2 is further provided with a spacer between the solid phase immobilizing part and the cleavage part, or (b) probe 1 is further provided with a spacer between the solid phase immobilizing part and the 3'-terminus of base sequence B1.
4. The probes for the detection of a target nucleic acid as described in Item 2, wherein either (a) probe 1 is further provided with a spacer between the solid phase immobilizing part and the cleavage part, or (b) probe 2 is further provided with a spacer between the solid phase immobilizing part and the 5'-terminus of base sequence B2.
5. The probes for the detection of a target nucleic acid as described either in Item 1 or in Item 3, wherein probe 2 is further provided with a labeling part between the 5'-terminus of base sequence B2 and the cleavage part. Here, the position at which the labeling part is placed is not limited to between the 5'-terminus of base sequence B2 and the cleavage part, but includes base sequence B2 within which its provision is made through various modes of bonding. The same applies to Items 13 and 21 as below.
6. The probes for the detection of a target nucleic acid as described either in Item 2 or in Item 4, wherein probe 1 is further provided with a labeling part between the 3'-terminus of base sequence B1 and the cleavage part. Here, the position at which the labeling part is placed is not limited to between the 3'-terminus of base sequence B1 and the cleavage part, but includes base sequence B1 within which its provision is made through various modes of bonding. The same applies to Items 14 and 22 as below.
7. The probes for the detection of a target nucleic acid as described in any of Items 1-6, wherein the cleavage part has a disulfide bond.
8. The probes for the detection of a target nucleic acid as described either in Item 5 or in Item 6, wherein the labeling part has digoxigenin.
   This invention also provides solid phases for the detection of a target nucleic acid as will be described the following Items 9-16.
9. A solid phase for the detection of a target nucleic acid comprising:
   (1) probe 1 having base sequence B1 complementary to A1 between two specific sequential base sequences A1 and A2 of the target nucleic acid, wherein (a) the 5'-terminus of base sequence B1 is phosphorylated and (b) a first solid phase immobilizing part is bound to the 3'-terminus of base sequence B1; and
   (2) probe 2 having base sequence B2 complementary to A2 between base sequences A1 and A2, wherein (a) the 5'-terminus of base sequence B2 is bound to one end of a cleavage part and (b) a second solid phase immobilizing part is bound to the other end of the cleavage part,
      wherein probe 1 is immobilized to a surface by the first solid phase immobilizing part and probe 2 is immobilized to the surface by the second solid phase immobilizing part.
10. A solid phase for the detection of a target nucleic acid comprising:
   (1) probe 1 having base sequence B1 complementary to A1 between two specific sequential base sequences A1 and A2 of the target nucleic acid, wherein (a) the 5'-terminus of base sequence B1 is phosphorylated, (b) the 3'-terminus of base sequence B1 is bound to one end of a cleavage part and (c) a first solid phase immobilizing part is bound to the other end of the cleavage part; and
   (2) probe 2 having base sequence B2 complementary to A2 between base sequences A1 and A2, wherein a second solid phase immobilizing part is bound to the 5'-terminus of base sequence B2,
      wherein probe 1 is immobilized to a surface by the first solid phase immobilizing part and probe 2 is immobilized to the surface by the second solid phase immobilizing part.
11. The solid phase for the detection of a target nucleic acid as described in Item 9, wherein either (a) probe 2 is further provided with a spacer between the second solid phase immobilizing part and the cleavage part, or (b) probe 1 is further provided with a spacer between the first solid phase immobilizing part and the 3'-terminus of base sequence B1.
12. The solid phase for the detection of a target nucleic acid as described in Item 10, wherein either (a) probe 1 is further provided with a spacer between the first solid phase immobilizing part and the cleavage part, or (b) probe 2 is further provided with a spacer between the second solid phase immobilizing part and the 5'-terminus of base sequence B2.
13. The solid phase for the detection of a target nucleic acid as described either in Item 9 or in Item 11, wherein probe 2 is further provided with a labeling part between the 5'-terminus of base sequence B2 and the cleavage part.
14. The solid phase for the detection of a target nucleic acid as described either in Item 10 or in Item 12, wherein probe 1 is further provided with a labeling part between the 3'-terminus of base sequence B1 and the cleavage part.
15. The solid phase for the detection of a target nucleic acid as described in any of Items 9-14, wherein the cleavage part has a disulfide bond.
16. The solid phase probes for the detection of a target nucleic acid as described either in Item 13 or in Item 14, wherein the labeling part has been labeled with digoxigenin.
   Further, this invention provides methods of detecting a target nucleic acid as will be described the following Items 17-25.
17. A method of detecting a target nucleic acid comprising:
   (A) a first step of immobilizing probe 1 to a surface of a solid phase through a first solid phase immobilizing part and immobilizing probe 2 to the surface of the solid phase through a second solid phase immobilizing part;
      wherein (1) probe 1 has base sequence B1 complementary to A1 between two specific sequential base sequences A1 and A2 of the target nucleic acid, further wherein (a) the 5'-terminus of base sequence B1 is phosphorylated and (b) the first solid phase immobilizing part is bound to the 3'-terminus of base sequence B1, and wherein (2) probe 2 has base sequence B2 complementary to A2 between base sequences A1 and A2, further wherein (a) the 5'-terminus of base sequence B2 is bound to one end of a cleavage part and (b) the second solid phase immobilizing part is bound to the other end of the cleavage part,
   (B) a second step of hybridizing probe 1, probe 2, and the target nucleic acid to form a hybrid;
   (C) a third step of ligating probe 1 and probe 2 in the hybrid by a ligase reaction;
   (D) a fourth step of cleaving the cleavage part of probe 2 by a cleavage reaction; and
   (E) a fifth step of detecting probe 1 which has been formed in the third step and which has bound base sequence B2 and the cleavage part of probe 2.
18. A method of detecting a target, nucleic acid comprising:
   (A) a first step of immobilizing probe 1 to a surface of a solid phase through a first solid phase immobilizing part and immobilizing probe 2 to the surface of the solid phase through a second solid phase immobilizing part;
      wherein (1) probe 1 has base sequence B1 complementary to A1 between two specific sequential base sequences A1 and A2 of the target nucleic acid, further wherein (a) the 5'-terminus of base sequence B1 is phosphorylated, (b) a cleavage part is bound to the 3'-terminus of base sequence B1, and (c) to the first solid phase immobilizing part is bound to the other end of the cleavage part, and wherein (2) probe 2 has base sequence B2 complementary to A2 between base sequences A1 and A2, further wherein the second solid phase immobilizing part is bound to the 5'-terminus of base sequence B2,
   (B) a second step of hybridizing probe 1, probe 2, and the target nucleic acid to form a hybrid;
   (C) a third step of ligating probe 1 and probe 2 in the hybrid by a ligase reaction;
   (D) a fourth step of cleaving the cleavage part of probe 1 by a cleavage reaction; and
   (E) a fifth step of detecting probe 2 which has been formed in the third step and which has bound base sequence B1 and the cleavage part of probe 1.
19. The method of detecting a target nucleic acid as described in Item 17, wherein either (a) probe 2 is further provided with a spacer between the second solid phase immobilizing part and the cleavage part, or (b) probe 1 is further provided with a spacer between the first solid phase immobilizing part and the 3'-terminus of base sequence B1.
20. The method of detecting a target nucleic acid as described in Item 18, wherein either (a) probe 1 is further provided with a spacer between the first solid phase immobilizing part and the cleavage part, or (b) probe 2 is further provided with a spacer between the second solid phase immobilizing part and the 5'-terminus of base sequence B2.
21. The method of detecting a target nucleic acid as described either in Item 17 or in Item 19, wherein probe 2 is further provided with a labeling part between the 5'-terminus of base sequence B2 and the cleavage part.
22. The method of detecting a target nucleic acid as described either in Item 18 or in Item 20, wherein probe 1 is further provided with a labeling part between the 3'-terminus of base sequence B1 and the cleavage part.
23. The method of detecting a target nucleic acid as described in any of Items 17-23, wherein the cleavage part has a disulfide bond and the disulfide bond is cleaved with a reducing agent in the fourth step.
24. The method of detecting a target nucleic acid as described in any of Items 17-22, wherein the mass of the probe bound to the solid phase is detected by surface plasmon resonance spectroscopy in the fifth step. Here, the mass of probe indicates the presence or the absence of probe 1 (or probe 2).
25. The method of detecting a target nucleic acid as described either in Item 21 or in Item 22, wherein the labeling part has been labeled with digoxigenin and the digoxigenin is detected in the fifth step.
   Still further, this invention provides methods of preparing a solid phase for the detection of a target nucleic acid as will be described the following Item 26-28.
26. A method of preparing a solid phase for the detection of a target nucleic acid, said method comprising:
   (A) a first step of hybridizing (1) two specific sequential base sequences A1 and A2 of the target nucleic acid, (2) probe 1 having base sequence B1 complementary to A1 between the two specific sequential base sequences A1 and A2 of the target nucleic acid, wherein (a) the 5'-terminus of base sequence B1 is phosphorylated and (b) a first solid phase immobilizing part is bound to the 3'-terminus of base sequence B1, and (3) probe 2 having base sequence B2 complementary to A2 between base sequences A1 and A2, wherein (a) the 5'-terminus of base sequence B2 is bound to one end of a cleavage part and (b) a second solid phase immobilizing part is bound to the other end of the cleavage part, to form a hybrid;
   (B) a second step of immobilizing the hybrid to a surface of a solid phase through the first and second solid phase immobilizing parts; and
   (C) a third step of removing the target nucleic acid from the hybrid.
27. A method of preparing a solid phase for the detection of a target nucleic acid, said method comprising:
   (A) a first step of hybridizing (1) two specific sequential base sequences A1 and A2 of the target nucleic acid, (2) probe 1 having base sequence B1 complementary to A1 between the two specific sequential sequences A1 and A2 of the target nucleic acid, wherein (a) the 5'-terminus of base sequence B1 is phosphorylated, (b) the 3'-terminus of base sequence B1 is bound to one end of a cleavage part, and (c) a first solid phase immobilizing part is bound to the other end of the cleavage part, and (3) probe 2 having base sequence B2 complementary to A2 between base sequences A1 and A2, wherein a second solid phase immobilizing part is bound to the 5'-terminus of base sequence B2, to form a hybrid;
   (B) a second step of immobilizing the hybrid to a surface of a solid phase through the first and second solid phase immobilizing parts; and
   (C) a third step of removing the target nucleic acid from the hybrid.
28. The method of preparing a solid phase for the detection of a target nucleic acid as described either in Item 26 or in Item 27, wherein either of probe 1 and probe 2 further contains either of or both of a spacer and a labeling part.

Hereinbelow, this invention will be explained in more detail by referring to embodiments thereof.

### BRIEF DESCRIPTION OF DRAWINGS

Fig. 1 is an illustration showing an example of the probes for the detection of a target nucleic acid according to this invention.
Fig. 2 is an illustration showing an example of the probes for the detection of a target nucleic acid according to the invention.
Fig. 3 is an illustration showing an example of the method of preparing a solid phase for the detection of a target nucleic acid according to the invention.
Fig. 4 is an illustration showing an example of the method of detecting a target nucleic acid according to the invention.
Fig. 5 is an illustration showing an example of the method of detecting a target nucleic acid according to the invention.
Fig. 6 is an illustration showing examples of the cleavage part of the probe for the detection of a target nucleic acid according to the invention.
Fig. 7 is an illustration showing examples of the cleavage part of the probe for the detection of a target nucleic acid according to the invention.
Fig. 8A is a schematic representation illustrative of the direct fluorescence detection technique, which is an embodiment of the method for the detection of a target nucleic acid according to the invention, as shown by Example 12.
Fig. 8B is a schematic representation illustrative of the direct fluorescence detection technique, which is an embodiment of the method for detecting a target nucleic acid according to the invention, as shown by Example 13.
Fig. 9 is a schematic representation illustrative of the digoxigenin-fluorescent antibody technique, which is an embodiment of the method of detecting a target nucleic acid according to the invention, as shown by Example 9.

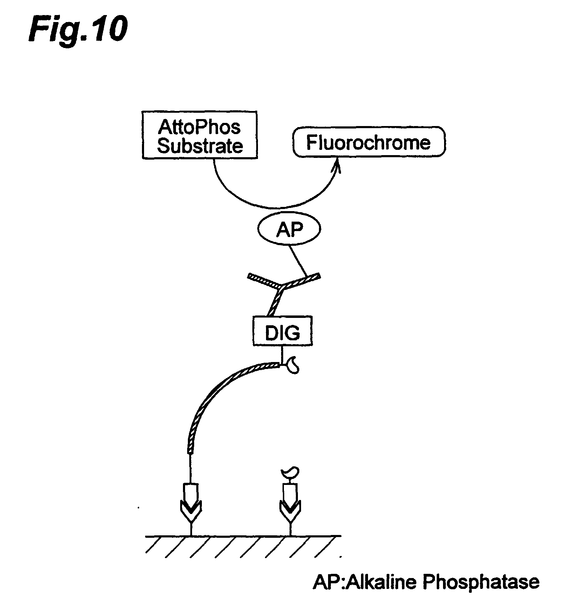

Fig. 10 is a schematic representation illustrative of the digoxigenin-enzyme antibody technique (fluorescence), which is an embodiment of the method of detecting a target nucleic acid according to the invention, as shown by Example 11.
Fig. 11 is a schematic representation illustrative of the digoxigenin-enzyme antibody technique (chemiluminescence), which is an embodiment of the method of detecting a target nucleic acid according to the invention, as shown by Example 14.

### BEST MODE FOR CARRYING OUT THE INVENTION

The probes for the detection of a target nucleic acid according to this invention comprises a pair of probes of two kinds (hereinafter referred to as "probe 1" and "probe 2"). Moreover, the solid phase for the detection of a target nucleic acid according to the invention is one in which the two kinds of probes according to the invention are immobilized on the surface of a solid phase while their adequate spatial arrangement is retained. Further, the method of detecting a target nucleic acid according to the invention detects the target nucleic acid with high sensitivity and high recognition using such a solid phase for the detection of a target nucleic acid. Still further, the method of preparing such solid phase for the detection of a target nucleic acid according to the invention is a method to prepare such solid phase for the detection of a target nucleic acid.

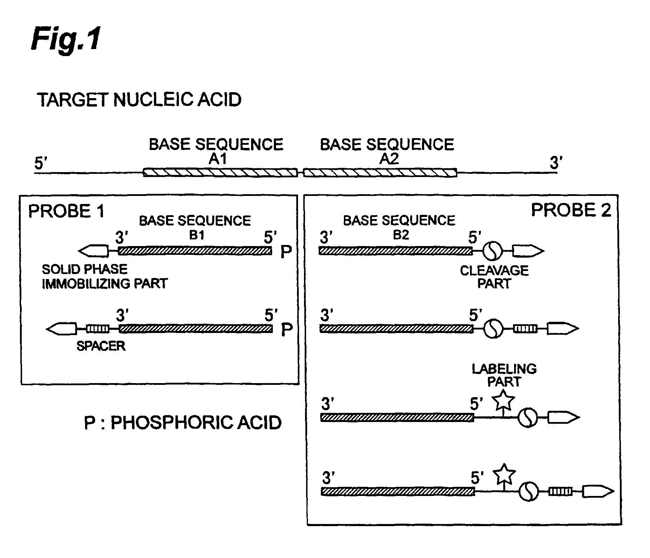

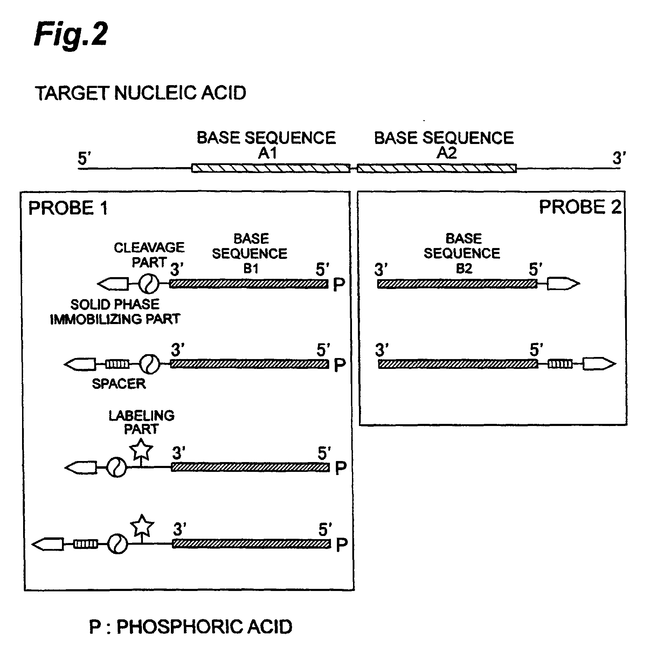

The actual structures of probe 1 and probe 2 according to this invention are shown in Fig. 1 (denoted "Type 1") and Fig. 2 (denoted "Type 2"), respectively. The structural difference between the probes shown in Figs. 1 and 2 is whether a cleavage part (or a labeling part, in addition) as will be illustrated below is to be introduced into probe 1 or is to be introduced into probe 2. Thus, in Type 1 shown in Fig. 1, the cleavage part (or the labeling part, in addition) is bound to the 5'-terminus of base sequence B2 of probe 2. On the other hand, in Type 2 shown in Fig. 2, the cleavage part (or the labeling part, in addition) is bound to the 3'-terminus of base sequence B1 of probe 1. Figs. 1 and 2 also show a target nucleic acid to be detected which has specific sequential (or continuous) base sequences A1 and A2 schematically. Probe 1 and probe 2 of Types 1 and 2 both have base sequences (B1 and B2) capable of sequentially hybridizing to A1 and A2. Further, the 5'-terminus of probe 1 is phosphorylated so that these probes can be ligated by ligase reaction (denoted "P" in the figures).

Moreover, there are no substantial differences between Type 1 and Type 2 in the manipulations for their detection of the target nucleic acid, their detection methods, and the results to be obtained. Explanation will then be made mainly on probe 1 and probe 2 of Type 1 (Fig. 1) in what follows.

Probes 1 and 2 have a solid phase immobilizing part (solid phase linker) that render them immobilized to the surface of a suitable solid phase. Such a solid phase immobilizing part will allow probe 1 and probe 2 to be capable of immobilization such that they occupy favorable spatial positions on the surface of the solid phase.

In addition, it is also possible that either probe 1 or probe 2 bears a spacer, if necessary, which will not be affected by hybridization, ligase reaction and cleavage reaction. Such a spacer is mainly used to adjust the distance from the surface of the solid phase so that the reaction immobilizing the probes to the solid phase of this invention and the detection reaction as will be explained below may proceed more efficiently.

Further, probe 2 bears a group (hereinafter referred to as "cleavage part") capable of being selectively cleaved by suitable chemical, enzymatic, or physical means.

Still further, probe 2 bears, if necessary, a labeling part that enables the combination with a variety of detection methods as will be explained below.

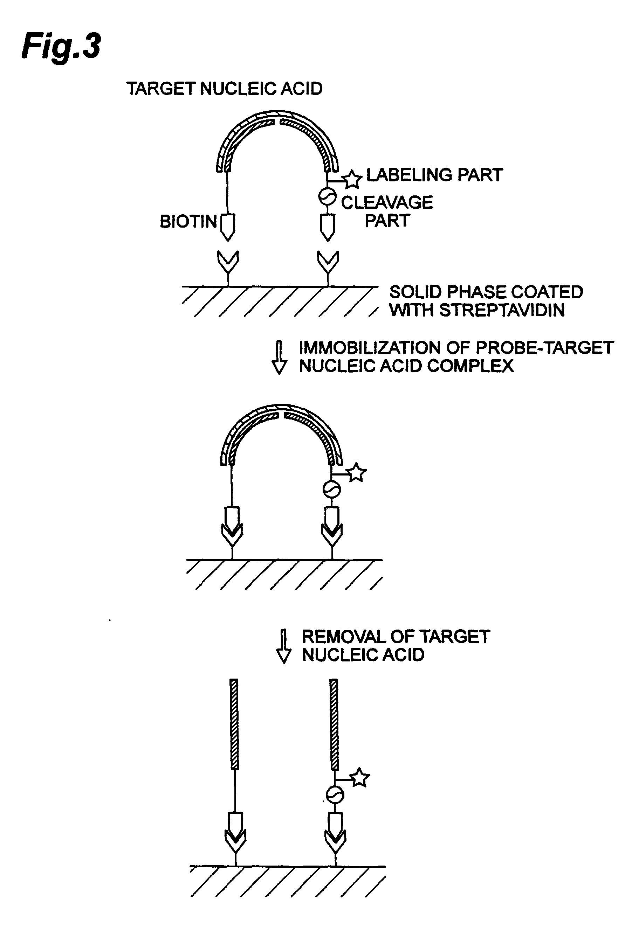

In addition, Fig. 3 schematically shows a preferred example of the solid phase for the detection of a target nucleic acid and the method of its preparation according to this invention. Through such method the spatial positions of probes 1 and 2, which are immobilized on the solid phase, allow the formation of a stable hybrid of the target nucleic acid with probe 1 and probe 2.

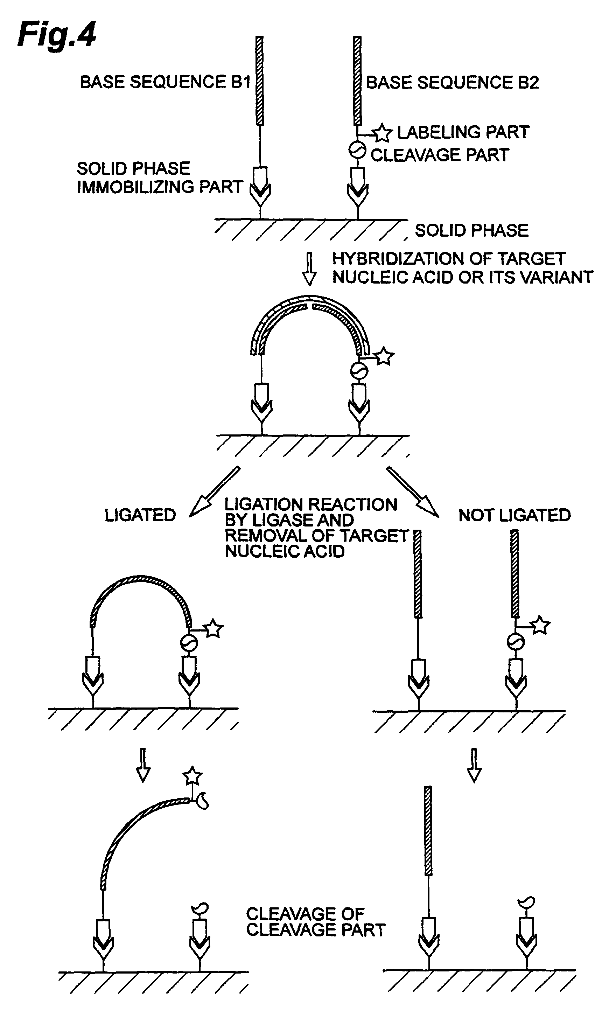

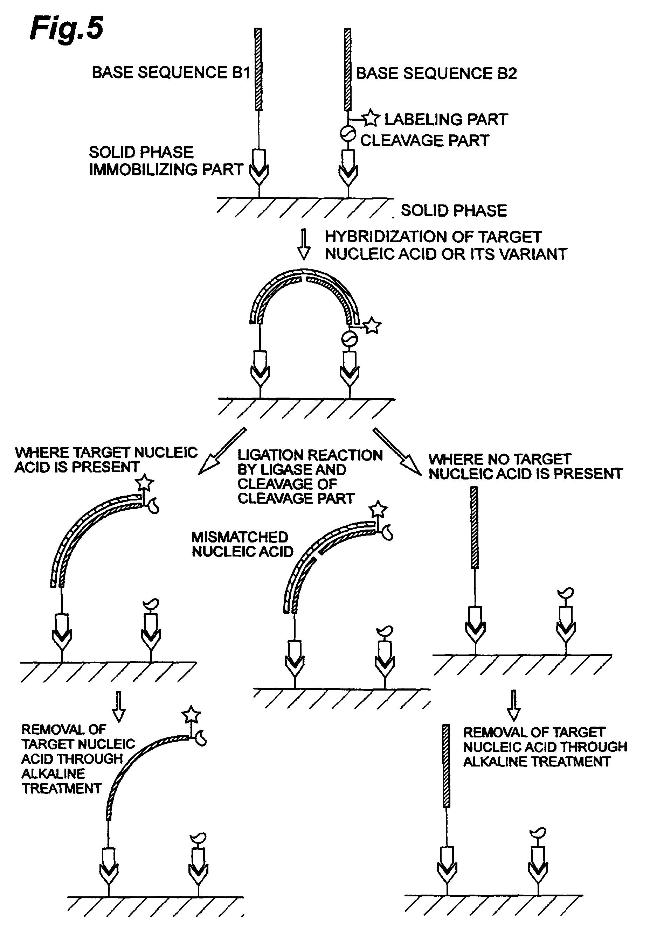

Furthermore, Figs. 4 and 5 schematically show examples of the methods of detecting a target nucleic acid by means of the solid phase for detection according to this invention. Specifically, when the solid phase for the detection of a target nucleic acid and the target nucleic acid are mixed, the target nucleic acid efficiently forms a hybrid with probe 1 and probe 2 on the surface of the solid phase. Then, probe 1 and probe 2 are ligated by a ligase reaction. At this juncture, only probe 1 and probe 2, which have formed the hybrid correctly, are allowed to be ligated due to the high recognition nature of the ligase reaction.

In Fig. 4, when the target nucleic acid is removed subsequently, the probes ligated by the ligase reaction will display a cyclic structure on the solid phase. Further, the probes that have not been ligated by the ligase reaction will return to their original state on the solid phase. Then, probe 2 is liberated directly from the solid phase by a reaction cleaving (or cutting) the cleavage part, while probe 2 ligated to probe 1 by the ligase reaction would not be liberated in solution at this point. On the other hand, probe 2 that has not been ligated to probe 1 by the ligase reaction is liberated in solution and will be removed. As a result, if probe 2 ligated by the ligase reaction remaining on the solid phase is detected, it means that the ligase reaction has occurred: namely, it indicates that the target nucleic acid has existed. Fig. 5 also schematically shows another example of the method of detecting a target nucleic acid by the use of the solid phase for detection according to the invention. Namely, in a similar manner to Fig. 4, there is shown a method in which after the formation of a hybrid and a ligase reaction are carried out, a reaction cleaving the cleavage part is first carried out and next, manipulations to remove the target nucleic acid are performed. In this method, Probe 2 ligated to probe 1 by the ligase reaction is not released in solution, whereas Probe 2 that has not been ligated to probe 1 is released in solution and will be eliminated similarly to Fig. 4. As a result, if probe 2 ligated by the ligase reaction remaining on the solid phase is detected, it means that the ligase reaction has occurred: namely, it indicates that the target nucleic acid has existed. A variety of techniques can be utilized in the method of detecting that probe 2 ligated by the ligase reaction remains on the solid phase. It is also possible to detect that probe 2 which has not been ligated by the ligase reaction. This case means that the ligase reaction has not occurred: it indicates that the target nucleic acid has never existed. A variety of techniques can be utilized in the method of detecting that probe 2, which has not been ligated by the ligase reaction, remains on the solid phase.

This invention will be hereinbelow explained in more detail.

### (Target Nucleic Acids)

In this invention, the types of nucleic acids (target nucleic acids) having specific polynucleotide sequences that can be detected according to the invention are not particularly limited, and a variety of nucleic acids (e.g., DNA, RNA, and oligonucleotides) are applicable. There is also no particular limitation concerning the length of the target nucleic acids and usable are the nucleic acids themselves or nucleic acids the length of which has been adjusted to appropriate sizes by suitable treatment depending on their intended purposes.

In addition, this invention employs two kinds of probes each having a base sequence capable of hybridizing with a specific polynucleotide sequence in the target nucleic acid to be detected. Therefore, this specific polynucleotide sequence needs to be previously known.

The base number of this specific polynucleotide sequence previously known is not particularly limited. However, the sequence is required to have a base number sufficient (1) to enable satisfactory hybridization with the probes of this invention and (2) to bind the probes through ligase reaction as will be described below. Such base number is dependent on the kinds and the base numbers of probes to be used, or on the type and the base number of the specific polynucleotide sequence of the target nucleic acid; but it is easy for those skilled in the art to find the optimum base number.

Concretely, the base number may sufficiently be at least 20 bases, and more preferably, not less than 30 bases. Additionally, if the base number grows too large, it is not preferable from the standpoint of synthesis of the probes, of the difficulty in their handling and the like.

As to the position of the specific polynucleotide sequence within the target nucleic acid, there is also no particular limitation; it may be in the vicinity of the termini or at the middle part of the nucleic acid.

Furthermore, when the method according to this invention is practiced, at least the specific polynucleotide sequence portion needs to be single-stranded; however, single-strandedness can readily be achieved by standard means in the art (e.g., heat or alkaline denaturation and treatment with a low concentration of salt).

### (Probes for Detection of Target Nucleic Acid)

As several examples of the probes according to this invention are illustrated in Fig. 1 (Type 1) and Fig. 2 (Type 2), they comprise a pair of probes of two kinds (probe 1 and probe 2). Type 1 will be explained: probe 2 is at least provided with a solid phase immobilizing part, a target recognition part, and a cleavage part; and probe 1 is at least provided with a solid phase immobilizing part and a target recognition part. Further, where necessary, probe 1 or probe 2 can bear a spacer. Still further, where necessary, probe 2 bears a labeling part for use in detection.

Probes 1 and 2 of Type 1 according to this invention can be constituted with respect to their structures concretely in what follows.
(I) The specific sequential base sequences A1 and A2 of a target nucleic acid to be detected is arbitrarily determined, as is shown in Fig. 1.
(II) Oligonucleotide B1 and oligonucleotide B2 that have base sequences complementary to base sequence A1 and base sequence A2 are determined, and they serve as base sequences for recognizing the base sequence in probes 1 and 2.
(III) The 5'-terminus of B1 of probe 1 is phosphorylated.
(IV) A solid phase immobilizing part is bound to the 3'-terminus of B1 of probe 1.
(V) Where necessary, a spacer is further linked between the 3'-terminus of B1 and the solid phase immobilizing part.
(VI) A solid phase immobilizing is bound to the 5'-terminus of B2 of probe 2 through a cleavage part.
(VII) Where necessary, a spacer is further linked between the cleavage part and the solid phase immobilizing part.
(VIII) Where necessary, a labeling part is further linked between the cleavage part and the 5'-terminus of B2.

Furthermore, the solid phase immobilizing part is not particularly limited insofar as it is a group capable of immobilizing said probes on the surface of a solid phase. The immobilization may be such that it is retained in the treatment for use in the method of detecting a target nucleic acid according to this invention. Concretely mentioned are bonding groups through ordinary chemical reactions, bonding groups through a variety of interactions, and the like. For the bonding groups through ordinary chemical reactions, the groups capable of forming covalent bonds with the activated groups (e.g., hydroxyl, carboxyl, and amino) of the surface of the solid phase. Concretely mentioned are ester, ether, amide and thioamide bonds. For the variety of interactions, bonding interaction between protein and ligand is mentioned, for example. Concretely, biotin-avidin interaction may preferably be chosen. In this case, it is preferred that avidin (or streptavidin) be immobilized to the surface of a solid phase in advance. Such a solid phase can be prepared according to standard methods known in the art. Moreover, there is no particular limitation to the method for introducing to the probes, said solid phase immobilizing parts; and standard bonding reactions can preferably be used. Specifically, the phosphoramidite method is applicable.

B1 and B2 of probes 1 and 2 that recognize the target nucleic acid have base sequences complementary to the specific polynucleotide sequence parts of the target nucleic acid and are able to sequentially hybridize with the specific polynucleotide sequence parts A1 and A2. There is no particular limitation to the base number of the base sequence of each probe, but the base number is about 10 or more, preferably 15 or more in this invention. If the base number is small, there will be no sufficient specific recognition function and the ligase reaction will also be difficult to be achieved. On the other hand, if it is too large, there will be problems in handling, preservation, or the like.

The cleavage part according to this invention is also not particularly limited insofar as it is a group capable of being selectively cleaved under a variety of suitable reaction conditions (chemical reactions, enzymatic reactions, and physicochemical reactions). Such selection can be made based on the conditions for the cleavage reaction, the groups of the cleavage part that are formed as a result of the cleavage reaction (e.g., whether they are the same groups or different groups), or the detection method utilizing a variety of labeling parts. The conditions for the cleavage reaction are not particularly limited, and standard chemical reactions, photoreactions or enzymatic reactions can be selected.

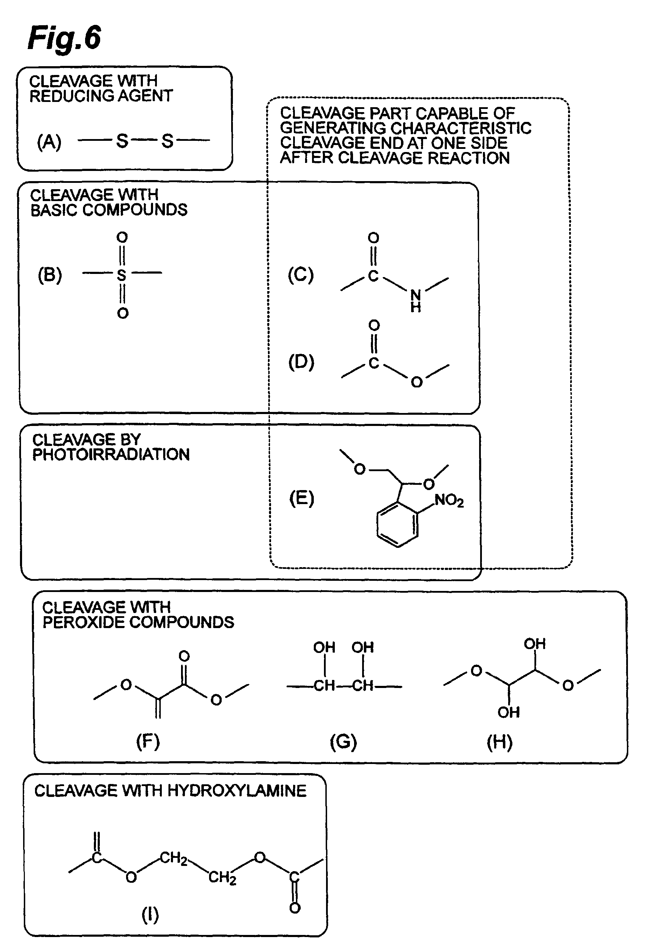

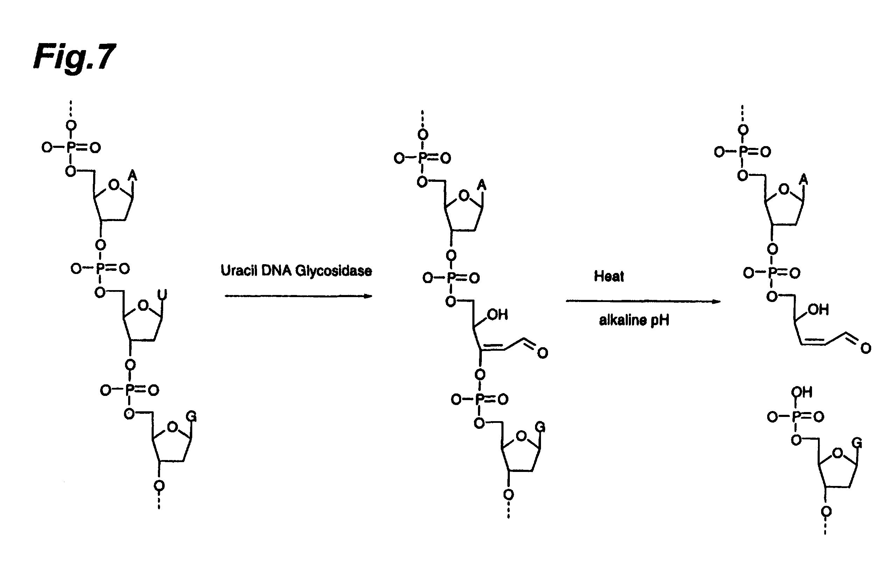

Several preferred examples of the cleavage part according to this invention are illustrated below by referring to Figs. 6(A)-6(I) and Fig. 7.
(A) Where the cleavage part is a disulfide bond (-S-S-).
   This case is preferable in that a variety of reducing agents is usable for the cleavage method. Dithiothreitol (denoted DTT) is concretely mentioned.
(B) Where the cleavage part is a -SO₂- bond.
   This case is preferable in that a variety of basic reagents is usable for the cleavage method.
(C), (D), (E) Where the cleavage part is an amide bond (including a thioamide bond, not shown in the figures), an ester bond, or an ether bond.
   In these cases, cleavage by a variety of chemical reactions, cleavage by photoreaction, cleavage by enzymatic reaction (e.g., peptidase and esterase) or the like is possible. Especially, in the cases of amide and ester bonds, hydrolysis may be mentioned; an ether bond of Type (E) can readily be cleaved by photoreaction. In addition, these cleavage parts of Types (C) (D) and (E) can generate characteristic cleavage ends at one side after the cleavage reaction. Therefore, only the characteristic cleavage ends can be labeled by a variety of reactions and can be used in detection.
(F), (G), (H) where the cleavage part has any of such bonding groups, generally it can readily be cleaved with peroxides. The peroxides include organic peroxides (e.g., peracetic acid) and inorganic peroxides (e.g., hydrogen peroxide).

(I) Where the cleavage part has such a bonding group, it can readily be cleaved with hydroxylamine.
   Furthermore, other examples will be specifically explained (although not shown in the figures).
   (i) Where the cleavage part is an RNA sequence.
      In this case, it is essential that the recognition part of the target nucleic acid be other than an RNA sequence such as a DNA sequence. In such case, cleavage with ribonuclease is possible.
   (ii) Where the cleavage part is a DNA sequence.
      In this case, it is essential that the recognition part of the target nucleic acid be other than a DNA sequence such as an RNA sequence. In such case, cleavage with deoxynuclease is possible.
   (iii) Where the cleavage part is a carbohydrate polymer.
      In this case, cleavage with a variety of glycosidases is possible.
   (iv) Where the cleavage part is deoxyuridine.
      Fig. 7 shows a cleavage method in the case where the cleavage part is deoxyuridine. Namely, its cleavage is feasible by heating under alkaline conditions subsequent to treatment with uracil DNA glycosidase: the cleavage generates cleavage ends one of which is aldehyde and the other of which is phosphoric acid. The uracil DNA glycosidase is one of DNA repair enzymes and catalyzes the reaction to remove uracil from a single- or double-stranded DNA which is formed by incorporation of dUTP or deamination of cytosine during the DNA replication process.

The labeling part according to this invention is a labeling group, which has been introduced to the probes described above, suitable for the combination with detection means. It aims at enabling the detection of the presence (or the absence) of such a labeling group on the solid phase after the cleavage reaction. Thus, said labeling group is not particularly limited, and various kinds of group that will achieve such a purpose can be selected for utilization. Also, the method for introducing such a labeling group is not particularly limited, and standard labeling techniques for nucleic acids known in the art are readily applicable.

Concretely, for the utilization of antigen-antibody reaction (i), there is mentioned labeling with digoxigenin. Methods that are ordinarily used are applicable in such labeling with digoxigenin. For the utilization of fluorescence (ii), there is mentioned labeling with fluorescent dyes (e.g., BODIPY493/503, Rhodamine Red, and TEXAS Red) and labeling with fluorescent beads. For the labeling with a fluorescent dye, its introduction can readily be done if the succinimide ester derivative of a fluorescent dye is allowed to react with an amino group, which has been introduced in advance, of an oligonucleotide to be used in the synthesis of probes; commercially available kits can be utilized for this purpose. For the labeling with fluorescent beads, it is feasible by chemically bonding the fluorescent beads to the probes. Furthermore, (iii) labeling with radioisotopes is mentioned. For example, in the synthesis of probes to which phosphoric acid groups have been introduced for ligase reaction, the probes can be labeled with radioisotopes by the use of ³²P.

In cases where mutually different groups are formed after the cleavage part has been cleaved (e.g., the cleavage parts of Types (C) (D) and (E) in Fig. 6)--as explained above--, the selective introduction of labels (i.e., labeling groups) for detection can further be done based on the chemical properties of the cleaved ends after cleavage.

If necessary, the respective probes can be further provided with spacers which will be explained below. By providing such a spacer, it becomes possible to aim at increasing the efficiency of hybridization as well as at optimizing and increasing the efficiency of various detection methods that can be employed in this invention, in addition to the cleavage reactions at various cleavage parts as explained above. Specifically, if a spacer of appropriate length is introduced between the solid phase immobilizing part and the cleavage part, cleavage reaction at the cleavage part can be carried out efficiently. Further, if a spacer is provided between the solid phase immobilizing part and base sequence B1 of probe 1, or it is provided between the solid phase immobilizing part and the cleavage part in probe 2, or both of the foregoing, then base sequences B1 and B2 in probes 1 and 2 that are capable of hybridizing to base sequences A1 and A2 of the target nucleic acid will be distanced from the surface of the solid phase. Because of this, it becomes possible to aim at increasing the efficiency of hybridization of the target nucleic acid to base sequences B1 and B2.

There is no particular limitation to the spacer that is preferably used here with respect to its type and shape. It may be sufficient insofar as it exerts enough strong binding ability in the following: the conditions where the two kinds of probes and the target nucleic acid are hybridized; manipulations for the accompanying washing; and other manipulations such as those for removal of the target nucleic acid. Concretely mentioned are an oligonucleotide, an oligopeptide, an alkylene chain, etc. Particularly, oligothymidine may preferably be used, and its required length can readily be obtained by the number of thymidine. An alkylene chain having an appropriate number of carbons can also preferably be used. The method for the introduction of such a spacer is not particularly limited, and a variety of banding reactions known in the art can be utilized. Concretely, it is easily feasible by utilizing the phosphoramidite method which is standard in oligonucleotide synthesis.

### (Solid Phases for Detection)

In this invention, the term "solid phase for detection" means a solid medium: the two kinds of probes explained above are bound to its surface adjacently with each other. Among others, its bonding density is not particularly limited and those bound with various densities can be used. In addition, the type of the solid medium is also not particularly limited and, for example, the solid media of inorganic substances or the solid media of organic substances can be used. As the solid medium of an inorganic substance, concretely mentioned are various metallic films (e.g., a sensor chip for use in a surface plasmon resonance sensor), silica gel, alumina, glass, quartz, etc. As the solid medium of an organic substance, concretely mentioned are nitrocellulose membranes, nylon membranes, plastic materials such as polystyrene and polyethylene, etc; it is also possible to use those in which carboxymethyl dextran is bound to the surfaces of the foregoing. In this invention, the use of a titer plate made of polystyrene or of quartz glass is preferred.

### (Methods of Preparing the Solid Phase)

According to this invention, the solid phase for detection is immobilized on a solid phase so that the two kinds of probes can have a predetermined spatial arrangement. Namely, it is the spatial arrangement that allows the probes to sequentially hybridize with the specific polynucleotide sequence of a target nucleic acid and then to be ligated by enzymatic reaction.

The methods to immobilize the probes with the predetermined spatial arrangement and to prepare the solid phase for detection include a technique wherein the two kinds of probes are premixed to give their desirable concentrations and the mixture is allowed to react with the solid phase for detection to achieve their bonding through the solid phase immobilizing parts, for example. In this case, there is obtained the two kinds of probes that are randomly bound to the surface of the solid phase immobilizing part. Here, it is believed that among the spatial arrangements by the two kinds of probes, only a very small number of the probes adopt that which allows the sequential hybridization with the specific polynucleotide sequence of the target nucleic acid and further the ligation by enzymatic reaction.

According to this invention, the means as described below can preferably be used as a method to immobilize as many pairs of probes as possible on the solid phase, which probes have the desirable spatial arrangement (Fig. 3). Namely, the two kinds of probes are first mixed with the target nucleic acid to effect hybridization. The resulting hybrid is allowed to cause bonding reaction on the solid phase for detection, which immobilizes the hybrid thereon. After thoroughly washing the solid phase, the target nucleic acid is removed from the hybrid by heat treatment, alkaline treatment or the like.

Following such manipulations, the two kinds of probes will be immobilized on the solid phase in such a desirable spatial arrangement as to be hybridized to the target nucleic acid.

### (Hybridization Conditions)

In this invention, there are no particular limitations to the conditions for hybridization between the pair of probes and the target nucleic acid according to the invention and standard conditions are usable. For example, the method as described in "Molecular Biology Experimental Manual"; M. Kawakami Ed.; Kodansha, pp. 172 or its modification may be used. Also, there are no particular limitations to the conditions under which the target nucleic acid is removed from the resulting hybrid to form a single-strand chain and standard conditions known in the art are preferably usable. For example, they are alkaline treatment, heat treatment, acid treatment and the like.

### (Enzymes)

In this invention, the enzymes which can be used to bind a pair of probes of the two kinds include a ligase, for example. The type and reaction conditions of ligase are not particularly limited and a variety of ligase reactions known in the art are usable in accordance with standard selections. For example, T4DNA ligase (available from Takara Shuzo), Ampligase (available from Epicentre Technologies Inc.), and Pfu DNA ligase (available from Stratagene Inc.) are preferably usable.

Further, after ligation resulting from the ligase reaction, it is possible to remove the target nucleic acid following various manipulations (e.g., heat treatment, alkaline treatment, and acid treatment).

### (Methods of Detection)

The methods of detecting a target nucleic acid according to this invention employ the solid phase for detection according to the invention; they allow the pair of probes on the solid phase for detection to sequentially hybridize with the specific polynucleotide sequence of the target nucleic acid, cause ligation between the two kinds of probes to take place through ligase reaction of the resulting hybrid, and utilize the resulting formation of a ligation product. In cases where the base sequence of the target nucleic acid differs and the probes experience a mismatch, the formation of a ligation product would not result from the ligase reaction. Thus after the target nucleic acid is removed by alkaline treatment or the like, the probes will not be ligated with each other and the respective ends remain to exist; the pair of probes will return to their initial state.

This invention further takes advantage of cleavage reaction of the cleavage part of the probe after the target nucleic acid has been removed by alkaline treatment or the like. In other words, where the ligation product is formed, the pair of probes according to the invention will remain on the solid phase in the state of being ligated by such a cleavage reaction. On the other hand, where the base sequence of the target nucleic acid differs and the probes experience a mismatch, the formation of the ligation product will not result from the ligase reaction. Thus after the target nucleic acid is removed by alkaline treatment or the like, the pair of probes according the invention will return to their initial state. Therefore, one member of the pair of probes according to the invention will be liberated in solution through cleavage reaction of the cleavage part and will not remain on the solid phase.

In the method of detecting a target nucleic acid according to this invention, a similar operation can be carried out by first allowing the cleavage reaction of the cleavage part of the probe after ligase reaction and by subsequently removing the target nucleic acid through alkaline treatment or the like. In this case, the pair of probes will likewise remain on the solid phase in the state of being ligated when the ligation product is formed by ligase reaction. In cases where the base sequence of the target nucleic acid differs and the probes experience a mismatch, one member of the pair of probes according to the invention will be released in solution and will not remain on the solid phase.

Accordingly, this invention finds out whether a ligase reaction has occurred by detecting the probes existing on the solid phase after the cleavage reaction, and based on the result, the invention makes it possible to determine the presence or the absence of the target nucleic acid.

Since the ligase reactions employed in this invention are known to possess extremely high base-specificity, the degree of mismatch can be kept to a very low level in the methods according to the invention.

For the method of detecting the structure of a probe existing on the solid phase after the cleavage reaction according to this invention, a variety of techniques known in the art can be used, as will be explained in the following.
(1) The labeling part bound to the ligation product is to be detected for its presence: antigens, enzymes, fluorescence, radioisotopes, etc. For example, where the labeling part is an antigen, it is recognized by an enzyme-bound antibody and the coloring of a substrate by the action of an enzyme is detected, which can ascertain the antigen.
(2) After the ligation reaction of probes, the cleavage part is cleaved. Then a labeled oligonucleotide, such as a fluorescent labeled oligonucleotide, is added for detection: the oligonucleotide has a chain that is complementary to the target nucleic acid recognizing part of the one probe containing the cleavage part or a chain that is complementary to a different base sequence within said probe. Such a technique is possible.
(3) If a decrease in the mass of the nucleic acid existing on the solid phase is detected by e.g., surface plasmon resonance spectroscopy, the disappearance of a probe can be confirmed.

This invention will be concretely explained hereinbelow by way of examples; however, the invention should not be limited to the following examples insofar as it does not depart from its essence.

Here, nucleic acids were generally synthesized on a nucleic acid synthesizer according to the solid phase phosphoramidite method and were purified by ion-exchange HPLC (purity greater than 99%).

5'-Phosphorylation employs 5'-Phosphate-ON Phosphoramidite, 5'-biotinylation employs Biotin Amidite, 3'-biotinylation employs Biotin-ON CPG, disulfide bond formation employs C6-Disulfide Phosphoramidite, and the introduction of an amino group within an oligonucleotide chain employs Uni-Link Amino Modifier. The foregoing reagents are available from Clontech. For the introduction of deoxyuridine, dU-CE Phosphoramidite (available from Glen Research) was employed.

Further, in carrying out the introduction of a fluorescent dye--BODIPY493/503, Rhodamine Red, or TEXAS Red--or antigen digoxigenin to oligonucleotides, BODIPY493/503 C3-SE, Rhodamine Red-X succinimidyl ester, TEXAS RED-X succinimidyl ester (each available from Molecular Probes) or Digoxigenin-3-O-methyl-carbonyl-ε -aminocaproic acid-N-hydroxysuccinimide ester (available from Boebringer Manheim) was allowed to react with the amino group that was introduced within an oligonucleotide chain by a known method.

### EXAMPLES

### (1) Disulfide Bond Cleavage Experiments for Probes in Solution

(1-1) Probe 2A comprising an oligonucleotide of 20 bases the 5'-terminus of which was biotinylated, 5'-(biotin)-GGTGGCGGCCGCTCTAGAAC-3', was automatically synthesized by a conventional phosphoramidite solid phase synthetic method.
(1-2) Probe 2B comprising an oligonucleotide of 22 bases the 5'-terminus of which was biotinylated and to which a disulfide bond (denoted [SS]) was introduced, 5'-(biotin)-TT-[SS]-GGTGGCGGCCGCTCTAGAAC-3', was automatically synthesized by the conventional phosphoramidite solid phase synthetic method.
(1-3) Probe 2C comprising an oligonucleotide of 25 bases the 5'-terminus of which was biotinylated and to which a disulfide bond was introduced, 5'-(biotin)-TTTTT-[SS]-GGTGGCGGCCGCTCTAGAAC-3', was automatically synthesized by the conventional phosphoramidite solid phase synthetic method.
(1-4) Probe 2D comprising an oligonucleotide of 30 bases the 5'-terminus of which was biotinylated and to which a disulfide bond was introduced, 5'-(biotin)-TTTTTTTTTT-[SS]-GGTGGCGGCCGCTCTAGAAC-3', was automatically synthesized by the conventional phosphoramidite solid phase synthetic method.
(1-5) The probes obtained in steps (1-1), (1-2), (1-3), and (1-4) (each 400 nM) were heated in 1xSSPE (150 mM NaCl, 10 mM NaH₂PO₄, 1 mM EDTA, pH 7.4) at 100 ° C for 5 min, respectively and cooled with ice immediately. To the respective probe solutions was added 1M dithiothreitol (abbreviated as "DTT") so as to give a concentration of 100 mM, and they were allowed to stand at 37 ° C for 10 min. When the DTT-probe solutions were allowed to react with BIAcore sensor chips (Pharmacia: SA5) coated with streptavidin, which would serve as solid phases, at 37 ° C for 5 min, the mass variations of the solid phases were observed with a surface plasmon resonance sensor (Pharmacia: BIAcore 2000). An increase of 1487 resonance units (which represents a value indicating an attenuation angle of reflected light in the surface plasmon resonance and which reflect the mass variation of a solid surface) was noted in probe 2A containing no disulfide bond. In contrast with this, an increase of 504 resonance units in probe 2B containing a disulfide bond, an increase of 588 resonance units in probe 2C containing a disulfide bond, and an increase of 935 resonance units in probe 2D containing a disulfide bond were observed. As compared to probe 2A containing no disulfide bond, the levels of immobilization for probes 2B, 2C and 2D each of which contained a disulfide bond were lower. This indicates that the disulfide bond has been cleaved with DTT in solution.

### (2) Disulfide Bond Cleavage Experiments for Probes on Solid Phase

(2-1) Immobilization of Probes
   The probes obtained in steps (1-1), (1-2), (1-3), and (1-4) (each 400 nM) were heated in 1xSSPE at 100 ° C for 5 min, respectively and cooled with ice immediately. After these probe solutions were allowed to react with BIAcore sensor chips coated with streptavidin at 37 ° C for 5 min, they were allowed to react with 0.1% aqueous sodium dodecyl sulfate solution, 10 mM aqueous sodium hydroxide solution, and 10 mM aqueous hydrochloric acid solution at 37 ° C for 1 min each in sequence to remove probes which were not bound to the solid phases.
(2-2) Reaction with DTT
   When the chips that immobilized the respective probes, as prepared in step (2-1), were allowed to react with 1xSSPE containing 100 mM DTT at 37 ° C for 10 min, the mass variations of the solid phases were observed with a surface plasmon resonance sensor. An increase of 31 resonance units in probe 2A containing no disulfide bond, a decrease of 544 resonance units in probe 2B containing a disulfide bond, a decrease of 495 resonance units in probe 2C containing a disulfide bond, and a decrease of 313 resonance units in probe 2D containing a disulfide bond were noted. Except for probe 2A, the resonance unit of each of probes 2B, 2C and 2D, which all contained a disulfide bond, decreased greatly. This indicates that the disulfide bond has experienced its cleavage by DTT on the solid phase.

### (3) Detection of Target Nucleic Acid (Surface Plasmon Resonance)

(3-1) Probe 1 comprising an oligonucleotide of 20 bases the 3'-terminus of which was biotinylated and the 5'-terminus of which was phosphorylated, 5'-(P)-TAGTGGATCCCCCGGGCTGC-(biotin)-3', was automatically synthesized by the conventional phosphoramidite solid phase synthetic method.
(3-2) For a target nucleic acid capable of retaining any of probes 2A, 2B, 2C and 2D and probe 1 obtained in step (3-1) adjacently, target nucleic acid A comprising an oligonucleotide of 40 bases with the sequence described below was synthesized.
   5'-GCAGCCCGGGGGATCCACTAGTTCTAGAGCGGCCGCCACC-3'
(3-3) Immobilization of Probes
   Probe 2B, probe 1 and target nucleic acid A (each 400 nM), or alternatively probe 2C, probe 1 and target nucleic acid A (each 400 nM) were mixed in 1xSSPE and heated at 100 ° C for 5 min to cause denaturation. Subsequently, they were maintained at 55 ° C for 10 min to effect hybridization. This hybrid was diluted 10-fold with 1xSSPE, and it was allowed to react with a BIAcore sensor chip coated with streptavidin at 37 ° C for 5 min, achieving the immobilization of probes. Then, it was allowed to react with 0.1% aqueous sodium dodecyl sulfate solution, 10 mM aqueous sodium hydroxide solution, and 10 mM aqueous hydrochloric acid solution at 37 ° C, respectively, for 1 min each in sequence. This caused target nucleic acid A to be dissociated from the probes.
(3-4) Hybridization of Target Nucleic Acid
   Target nucleic acid A (400 nM) was allowed to react with the solid phases, from which target nucleic acid A having retained the two probes adjacently in step (3-3) had been dissociated, in 1xSSPE at 37 ° C for 5 min.
(3-5) Ligase Reaction on Solid Phase
   The solid phases that hybridized target nucleic acid A in step (3-4) were allowed to react with T4 DNA ligase diluted with the reaction buffer as attached (which contained 3500 IU ligase per 1 ml: Takara Shuzo) at 37 ° C for 5 min. Subsequently, they were allowed to react with 0.1% aqueous sodium dodecyl sulfate solution, 10 mM aqueous sodium hydroxide solution, and 10 mM aqueous hydrochloric acid solution at 37 ° C for 1 min in sequence. This caused target nucleic acid A to be dissociated from the probes.
(3-6) Cleavage of the Disulfide Bond by DTT
   When the solid phases prepared in step (3-5) were allowed to react with 1xSSPE containing 100 mM DTT at 37 ° C for 10 min, the mass variations of the solid phases were observed with a surface plasmon resonance sensor. A decrease of 67 resonance units in probe 2B/probe 1 and a decrease of 71 resonance units in probe 2C/probe 1 were noted. On the other hand, with respect to the solid phases that were subjected to T4 DNA ligase without being hybridized to target nucleic acid A, a decrease of 136 resonance units in probe 2B/probe 1 and a decrease of 131 resonance units in probe 2C/probe 1 were noted. As compared to the solid phases with probe 2B/probe 1 and probe 2C/probe 1 that had not been hybridized to target nucleic acid A, decreases in resonance unit of those which had been hybridized to target nucleic acid A were suppressed. In a combination of probe 2B and probe 1, or of probe 2C and probe 1, both the probes were hybridized to target nucleic acid A and were retained adjacently, and they were ligated by T4 DNA ligase. Therefore, even when they experienced cleavage of the disulfide bond of probe 2B or probe 2C by the action of DTT, the sequence recognizing the target nucleic acid in probe 2B or probe 2C remained on the solid phases without being washed away, and large variations in mass on the solid phases did not take place. The foregoing results indicate that the present method enables the detection of a target nucleic acid.

### (4) Disulfide Bond Cleavage Experiments for Probe Labeled with Fluorescent Dye in Solution

(4-1) Probe 2E comprising an oligonucleotide of 20 bases the 5'-terminus of which was biotinylated and to which a disulfide bond and a fluorescent dye, BODIPY493/503 (denoted "F") were introduced, 5'-(biotin)-[SS]-F-GGTGGCGGCCGCTCTAGAAC-3', was automatically synthesized by the conventional phosphoramidite solid phase synthetic method.
(4-2) Probe 2A and probe 2E (each 400 nM) were heated in 1xSSPE at 100 ° C for 5 min and cooled with ice immediately. To the respective probe solutions was added 1M DTT so as to give a concentration of 100 mM and they were allowed to stand at 37 ° C for 10 min. When the DTT-probe solutions were allowed to react with BIAcore sensor chips coated with streptavidin at 37 ° C for 5 min, the mass variations of the solid phases were observed with a surface plasmon resonance sensor. An increase of 1377 resonance units was noted in probe 2A containing no disulfide bond, whereas the increase of probe 2E containing a disulfide bond remained at only 103 resonance units. When probe 2E was allowed to react with 1 mM DTT, an increase of 717 resonance units was noted; and the higher the DTT concentration was, smaller the level of immobilization became. These results indicate that the disulfide bond of probe 2E has been cleaved with DTT in solution.

### (5) Disulfide Bond Cleavage Experiment for Probe Labeled with Fluorescent Dye on Solid Phase

(5-1) Preparation of a Sensor Chip Coated with Avidin
   A solution of 50 mM N-hydroxysuccinimide (SIGMA) containing 200 mM 1-ethyl-3-(3-dimethylaminopropyl)carbodiimide (SIGMA) was allowed to react with a BIAcore sensor chip (Pharmacia: CM5) bound to carboxymethyldextran in a surface plasmon resonance sensor at 37 ° C for 7 min. Subsequently, it was allowed to react with 10 mM acetic acid buffer (pH 5.0) containing avidin (100 µg per ml: SIGMA) at 37 ° C for 1-8 min. The reaction was quenched by adding 1 M ethanolamine hydrochloride the pH of which had been adjusted to 8.5 with aqueous sodium hydroxide solution at 37 ° C for 7 min.
(5-2) Immobilization of the Probe
   Probe 2E (400 nM) was heated in 1xSSPE at 100 ° C for 5 min and cooled with ice immediately. After this probe solution was allowed to react with the BIAcore sensor chip coated with avidin, which had been prepared in step (5-1), at 37 ° C for 5 min, it was washed with 0.1% aqueous sodium dodecyl sulfate solution, 10 mM aqueous sodium hydroxide solution, and 10 mM aqueous hydrochloric acid solution, respectively, at 37 ° C for 1 min each in sequence.
(5-3) Reaction with DTT
   The chip that immobilized probe 2E, as prepared in step (5-2) and 1xSSPE containing 100 mM DTT were reacted at 37 ° C for 10 min. The chip was washed with 0.1% aqueous sodium dodecyl sulfate solution, 10 mM aqueous sodium hydroxide solution, and 10 mM aqueous hydrochloric acid solution, respectively, at 37 ° C for 1 min each in sequence. Then, the mass variation of the solid phase was observed with a surface plasmon resonance sensor. A decrease of 1400-2100 resonance units was noted. This result indicates that the disulfide bond of probe 2E has been cleaved with DTT on the solid phase.

### (6) Detection of Target Nucleic Acid Using Probe Labeled with Fluorescent Dye (Surface Plasmon Resonance)

(6-1) Preparation of Probes
   A one base excessive sequence comprising an oligonucleotide of 41 bases obtainable from the insertion of adenosine between the 20th base and the 21st base from the 5'-terminus of target nucleic acid A, 5'-GCAGCCCGGGGGATCCACTAAGTTCTAGAGCGGCCGCCACC-3', was automatically synthesized by the conventional phosphoramidite solid phase synthetic method.
(6-2) A one base deficient sequence comprising an oligonucleotide of 39 bases obtainable from the deletion of adenosine at the 20th base from the 5'-terminus of target nucleic acid A, 5'-GCAGCCCGGGGGATCCACTGTTCTAGAGCGGCCGCCACC-3', was automatically synthesized by the conventional phosphoramidite solid phase synthetic method.
(6-3) Preparation of a Sensor Chip Coated with Avidin
   A solution of 50 mM N-hydroxysuccinimide containing 200 mM 1-ethyl-3-(3-dimethylaminopropyl)carbodiimide was allowed to react with a BIAcore sensor chip bound to carboxymethyldextran in a surface plasmon resonance sensor at 37 ° C for 7 min. Subsequently, it was allowed to react with 10 mM acetic acid buffer (pH 5.0) containing avidin (5 µg per ml) at 37 ° C for 1-8 min. The reaction was quenched by adding 1 M ethanolamine hydrochloride the pH of which had been adjusted to 8.5 with aqueous sodium hydroxide solution at 37 ° C for 7 min.
(6-4) Immobilization of Probes
   Probe 2E, probe 1, and target nucleic acid A (each 400 nM) were mixed in 1xSSPE and heated at 100 ° C for 5 min to cause denaturation. Subsequently, they were maintained at 55 ° C for 10 min to effect hybridization. This hybrid was diluted 10-fold with 1xSSPE, and it was allowed to react with the BIAcore sensor chip coated with avidin, which was prepared in step (6-3), at 37 ° C for 5 min, achieving the immobilization of the probes. Then, it was allowed to react with 0.1% aqueous sodium dodecyl sulfate solution, 10 mM aqueous sodium hydroxide solution, and 10 mM aqueous hydrochloric acid solution, respectively, at 37 ° C for 1 min each in sequence. This caused target nucleic acid A to be dissociated from the probes.
(6-5) Hybridization of Target Nucleic Acid A and Variant Nucleic Acids Thereof
   Target nucleic acid A, the one base excessive sequence, and the one base deficient sequence--variants of target nucleic acid A--(each 400 nM) were allowed to react with the solid phases (at different locations), from which the target nucleic acid A having retained the two probes adjacently in step (6-4) had been dissociated, in 1xSSPE at 37 ° C for 5 min. When the mass variations of the solid phases were observed with a surface plasmon resonance sensor, increases of 337-379 resonance units were noted. Thus it was ascertained that the solid phases prepared in step (6-4) hybridized with target nucleic acid A and its variant nucleic acids (i.e., the one base excessive sequence and the one base deficient sequence). This indicates that either of the sequences binds to the solid phase and that the nucleic acid sequence to be detected can not be distinguished from its slightly different base sequences based on the bonding quantities due to hybridization. When none of the target nucleic acids was hybridized to the solid phase, the increase remained at only 6 resonance units.
(6-6) Ligase Reaction on Solid Phase
   The solid phases that were hybridized to target nucleic acid A, the one base excessive sequence and the one base deficient sequence-- variant nucleic acids of thereof--in step (6-5) were allowed to react with T4 DNA ligase diluted with a reaction buffer as attached (which contained 3500 IU ligase per 1 ml) at 37 ° C for 5 min. Subsequently, they were allowed to react with 0.1% aqueous sodium dodecyl sulfate solution, 10 mM aqueous sodium hydroxide solution, and 10 mM aqueous hydrochloric acid solution, respectively, at 37 ° C for 1 min each in sequence. This caused target nucleic acid A and its variant nucleic acids to be dissociated from the probes.
(6-7) Cleavage of the Disulfide Bond by DTT
   When the solid phases prepared in Step (6-6) were allowed to react with 1xSSPE containing 100 mM DTT at 37 ° C for 10 min, the mass variations of the solid phases were observed with a surface plasmon resonance sensor. A decrease of 82 resonance units in the solid phase that allowed hybridization of the one base deficient sequence, a decrease of 95 resonance units in the solid phase that allowed hybridization of the one base excessive sequence, and a decrease of 116 resonance units in the solid phase that did not allow hybridization of any of the target nucleic acids were noted. On the other hand, the decrease in the solid phase that allowed hybridization of target nucleic acid A remained at 54 resonance units. As compared to the other solid phases, the decrease in resonance unit of the one that allowed hybridization of target nucleic acid A was suppressed for the following reason. Both of probe 2E and probe 1 were hybridized to target nucleic acid A and were retained adjacently, and they were ligated by T4 DNA ligase. Therefore, even when they were subjected to cleavage of the disulfide bond of probe 2E by the action of DTT, the sequence recognizing the target nucleic acid in probe 2E remained on the solid phase without being washed away, and a large variation in mass of the solid phase did not take place. These results indicate that the difference of one base in the sequence of a target nucleic acid around the ligation of probes has been recognized.

### (7) Cleavage Experiments for Probes Labeled with Digoxigenin

(7-1) Preparation of Probes
Probe 2F comprising an oligonucleotide of 22 bases the 5'-terminus of which was biotinylated and the oligonucleotide chain of which was subjected to digoxigenin modification (in the present specification or the drawings, it may sometimes be denoted [DIG]) where two thymidine bases were introduced between the biotin and the digoxigenin, 5'-(biotin)-TT-[DIG]-GGTGGCGGCCGCTCTAGAAC-3', was automatically synthesized by the conventional phosphoramidite solid phase synthetic method.
(7-2) Probe 2G comprising an oligonucleotide of 22 bases the 5'-terminus of which was biotinylated and the oligonucleotide chain of which was subjected to disulfide bond and digoxigenin modification where two thymidine bases were introduced between the biotin and the disulfide bond, 5'-(biotin)-TT-[SS]-[DIG]-GGTGGCGGCCGCTCTAGAAC-3', was automatically synthesized by the conventional phosphoramidite solid phase synthetic method.
(7-3) Immobilization of Probes
Probe 2F and probe 2G (each 500 pM) were heated in 1xSSPE at 95 ° C for 5 min and cooled with ice immediately. Solutions of these probes were added to a 96-well microtiter plate coated with streptavidin as attached to a NORTHERN ELISA (digoxigenin detection ELISA kit available from Boehringer Manheim) at 94 µl per well. With agitating reaction was allowed to proceed at 37 ° C for 30 min.
(7-4) Cleavage of the Disulfide Bond by DTT
After the respective wells were three times washed with 250 µl of a washing solution attached to the NORTHERN ELISA, 1xSSPE containing each concentration of l M, 100 mM, and 1 mM DTT was added to the wells at 100 µl per well. With agitating reaction was allowed to proceed at 37 ° C for 30 min.
(7-5) Detection of Digoxigenin
After the respective wells were three times washed with 250 µl of the washing solution, reaction was allowed to proceed at 37 ° C for 30 min while being agitated with Anti-DIG-POD (anti-digoxigenin antibody conjugated with peroxidase: 100 µl containing 5 mU antibody per well added). After the wells were three times washed with 250 µl of the washing solution, they were allowed to react with a TMB Substrate Solution attached to the NORTHERN ELISA (peroxidase substrate trimethylbenzidine: 100 µl per well added) at room temperature for 5 min. The reaction was quenched with a Stop Reagent attached to the NORTHERN ELISA (100 µl per well added). Trimethylbenzidine turns into a product developing blue color by the action of peroxidase, which product is then amplified. Addition of the Stop Reagent terminates the amplification reaction of the compound developing blue color and the solution displays yellow. The quantity of peroxidase within the well, then the quantity of digoxigenin can be estimated by the magnitude of light absorbance at wavelengths of from 450 nm to 500 nm. Absorbance at 470 nm was measured with a microplate reader, SPECTRA MAX 250 (Molecular Devices); the results are shown in the following table (the mean values of two wells).

| DTT concentration | 1 M | 100 mM | 1 mM | 0 mM |
|---|---|---|---|---|
| probe 2F | 2.82 | 3.26 | 3.22 | 3.30 |
| probe 2G | 0.03 | 0.12 | 2.12 | 2.99 |

A greater value means that more digoxigenin is present in the well of the microtiter plate. From the above table, it was observed that in probe 2F containing no disulfide bond absorbance at 470 nm did not change with varying concentrations of DTT, whereas in probe 2G containing a disulfide bond absorbance at 470 nm changed with varying concentrations of DTT. These results indicate that the disulfide bond is cleaved with DTT on the solid phase.

### (8) A Comparison of Efficiency in Disulfide Bond Cleavage between the Presence and the Absence of a Spacer Introduced between Biotin and the Disulfide Bond

(8-1) Preparation of Probes
Probe 2H comprising an oligonucleotide of 20 bases the 5'-terminus of which was biotinylated and the oligonucleotide chain of which was subjected to disulfide bond and digoxigenin modification where no thymidine base was introduced between the biotin and the disulfide bond, 5'-(biotin)-[SS]-[DIG]-GGTGGCGGCCGCTCTAGAAC-3', was automatically synthesized by the conventional phosphoramidite solid phase synthetic method.
(8-2) Probe 2I comprising an oligonucleotide of 25 bases the 5'-terminus of which was biotinylated and the oligonucleotide chain of which was subjected to disulfide bond and digoxigenin modification where five thymidine bases were introduced between the biotin and the disulfide bond, 5'-(biotin)-TTTTT-[SS]-[DIG]-GGTGGCGGCCGCTCTAGAAC-3', was automatically synthesized by the conventional phosphoramidite solid phase synthetic method.
(8-3) Probe 2J comprising an oligonucleotide of 30 bases the 5'-terminus of which was biotinylated and the oligonucleotide chain of which was subjected to disulfide bond and digoxigenin modification where 10 thymidine bases were introduced between the biotin and the disulfide bond, 5'-(biotin)-TTTTTTTTTT-[SS]-[DIG]-GGTGGCGGCCGCTCTAGAAC-3', was automatically synthesized by the conventional phosphoramidite solid phase synthetic method.
(8-4) Immobilization of Probes
Probe 2G, probe 2H, probe 2I, and probe 2J (each 500 pM) were heated in 1xSSPE at 95 ° C for 5 min and cooled with ice immediately. Solutions of these probes were added to a 96-well microtiter plate coated with streptavidin as attached to a NORTHERN ELISA at 94 µl per well. With agitating reaction was allowed to proceed at 37 ° C for 30 min.
(8-5) Cleavage of the Disulfide Bond by DTT
After the respective wells were three times washed with 250 µl of a washing solution attached to the NORTHERN ELISA, 1xSSPE containing DTT (500 mM, 100 mM, and 0 M) and 0.1% sodium dodecyl sulfate was added to the wells at 100 µl per well. With agitating reaction was allowed to proceed at 37 ° C for 30 min.
(8-6) Detection of Digoxigenin
After the respective wells were three times washed with 250 µl of the washing solution, reaction was allowed to proceed at 37 ° C for 30 min while being agitated with Anti-DIG-POD (100 µl containing 5 mU antibody per well added). After the respective wells were three times washed with 250 µl of the washing solution, they were allowed to react with a TMB Substrate Solution attached to the NORTHERN ELISA (100 µl per well added) at room temperature for 5 min. The reaction was quenched with a Stop Reagent attached to the NORTHERN ELISA (100 µl per well added). Absorbance at 490 nm was measured with a microplate reader, SPECTRA MAX 250; the results are shown in the table below. The values represent the quantities of digoxigenin remaining on the solid phases which are expressed as residual percentages when absorbance at 490 nm was made 100 where the probes were subjected to treatment at a DTT concentration of 0 M (the mean values of two wells).

| DTT concentration | 500 mM | 100 mM | 0 M |
|---|---|---|---|
| probe 2H (thymidine: 0) | 27.5 | 75.0 | 100 |
| probe 2G (thymidine: 2) | 4.4 | 24.0 | 100 |
| probe 2I (thymidine: 5) | 3.9 | 21.4 | 100 |
| probe 2J (thymidine: 10) | 4.3 | 19.1 | 100 |

In the probes (2G, 2I, and 2J) where two or more thymidine bases had been introduced to the biotin and the disulfide bond, the respective percentages were around 20% at DTT concentrations of 100 mM, and around 4% at 500 mM. On the other hand, probe 2H gave about a threefold greater value (75%) at a DTT concentration of 100 mM, and about a sixfold greater value at a DTT concentration of 500 mM. This indicates that cleavage of the disulfide bond has not proceeded smoothly because an adequate spacer was provided between the biotin and the disulfide bond. From this result, it is preferred that a spacer be introduced between the disulfide bond and the biotin, which is a solid phase immobilizing part, in order to improve the efficiency of cleavage reaction of the disulfide bond which is the cleavage part of a probe on the solid phase.

### (9) Detection of Target Nucleic Acid Using Probe Labeled with Digoxigenin (Enzyme-Labeled Antibody Technique (colorimetry))

(9-1) Immobilization of Probes
Probe 1, probe 2G, and target nucleic acid A (each 300 pM) were mixed in 1xSSPE and heated at 95 ° C for 5 min to cause denaturation. Subsequently, they were maintained at 55 ° C for 10 min to effect hybridization. This hybrid was added to a 96-well microtiter plate coated with streptavidin as attached to a NORTHERN ELISA at 94 µl per well. With agitating reaction was allowed to proceed at 37 ° C for 30 min. After the respective wells were three times washed with 250 µl of a washing solution attached to the NORTHERN ELISA, 15 mM aqueous sodium hydroxide solution was added to the wells at 100 µl per well. The reaction was allowed to proceed at 37 ° C for 30 min, causing target nucleic acid A to be dissociated from the probes.
(9-2) Hybridization of Target Nucleic Acid A and Variant Nucleic Acids Thereof
To the respective wells that immobilized the probes in step (9-1) were added target nucleic acid A, the one base excessive sequence, and the one base deficient sequence--variant nucleic acids thereof--(each 600 nM) at 100 µl per well. After allowing to react in 1xSSPE at 37 ° C for 30 min, the wells were three times washed with 250 µl of the washing solution.
(9-3) Ligase Reaction on Solid Phase
To the wells prepared in step (9-2) was added T4 DNA ligase diluted with a reaction buffer as attached (which contained 30 IU ligase per well), and they were allowed to react at 37 ° C for 30 min. After washing the wells with the washing solution three times, the wells were allowed to react with 20 mM aqueous sodium hydroxide solution at 37 ° C for 30 min. This caused target nucleic acid A and its variant nucleic acids (i.e., the one base excessive sequence and the one base deficient sequence) to be dissociated from the probes.
(9-4) Cleavage of the Disulfide Bond by DTT
To the wells prepared in step (9-3) was added 1xSSPE containing 1 M DTT at 100 µl per well. After allowing to react at room temperature for 30 min, the respective wells were three times washed with 250 µl of the washing solution and once with 100 µl of 0.1% aqueous sodium dodecyl sulfate solution.
(9-5) Detection of Digoxigenin
The wells prepared in step (9-4) were allowed to react with Anti-DIG-POD (100 µl containing 5 mU antibody per well added) at 37 ° C for 30 min with shaking. After washing the wells with 250 µl of the washing solution four times, they were allowed to react with a TMB Substrate Solution attached to the NORTHERN ELISA (100 µl per well added) at room temperature for 10 min. The reaction was quenched with a Stop Reagent attached to the NORTHERN ELISA (100 µl per well added). Absorbance at 490 nm was measured with a microplate reader, SPECTRA MAX 250; the results are shown in the table below (the mean of three wells±SD values).

| target nucleic acid A | one base excessive sequence | one base deficient sequence | no addition of nucleic acid |
|---|---|---|---|
| 0.861±0.077 | 0.182±0.052 | 0.140±0.019 | 0.137±0.052 |

The one base excessive sequence and the one base deficient sequence, which were both variant nucleic acids, showed values similar to that in the case of no addition of nucleic acid, and only target nucleic acid A showed a significantly large value. This indicates that the difference of one base in the sequence of the target nucleic acid around the ligation of probes has been recognized. From these experimental results, it has been demonstrated that the presence or the absence of a target nucleic acid can be detected.

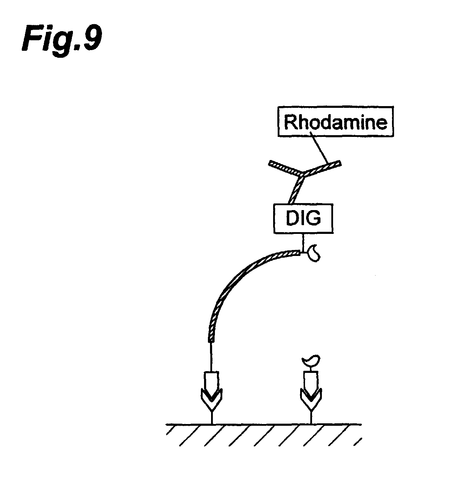

### (10) Detection of Target Nucleic Acid Using Probe Labeled with Digoxigenin (Fluorescent Antibody Technique, Fig. 9)

(10-1) Immobilization of Probes
Probe 1, probe 2G, and target nucleic acid A (each 500 nM) were mixed in 1xSSPE and heated at 95 ° C for 5 min to cause denaturation. Subsequently, they were maintained at 55 ° C for 10 min to effect hybridization. This hybrid was added to a 96-well microtiter plate in black coated with streptavidin (Boehringer Manheim) at 94 µl per well. With agitating reaction was allowed to proceed at 37 ° C for 30 min. After the respective wells were three times washed with 250 µl of a washing solution attached to a NORTHERN ELISA, 10 mM aqueous sodium hydroxide solution containing 0.1% sodium dodecyl sulfate was added to the wells at 100 µl per well. The reaction was allowed to proceed at 37 ° C for 10 min, causing target nucleic acid A to be dissociated from the probes.
(10-2) Hybridization of Target Nucleic Acid A and Variant Nucleic Acids Thereof
To the respective wells that had immobilized the probes in step (10-1) were added target nucleic acid A, the one base excessive sequence and the one base deficient sequence--variant nucleic acids thereof--(each 1 µM) at 100 µl per well. After allowing to react in 1xSSPE at 37 ° C for 30 min, the wells were three times washed with 250 µl of the washing solution.
(10-3) Ligase Reaction on Solid Phase
To the wells prepared in step (10-2) was added T4 DNA ligase diluted with a reaction buffer as attached (which contained 30 IU ligase per well), and they were allowed to react at 37 ° C for 30 min. After washing the wells with the washing solution three times, 10 mM aqueous sodium hydroxide solution containing 0.1% sodium dodecyl sulfate was added to the wells at 100 µ l per well. This caused target nucleic acid A and its variant nucleic acids to be dissociated from the probes.
(10-4) Cleavage of the Disulfide Bond by DTT
To the wells prepared in step (10-3) was added 1xSSPE containing 500 mM DTT and 0.1% sodium dodecyl sulfate at 100 µl per well. After allowing to react at room temperature for 30 min, the respective wells were three times washed with 250 µl of the washing solution.
(10-5) Reaction with Rhodamin-Labeled anti-DIG Antibody
To the wells prepared in step (10-4) was added Rhodamin-labeled anti-digoxigenin antibody (Boehringer Manheim: 100 µl containing 2 µg antibody per well). After allowing to react at 37 ° C for 30 min, the respective wells were three times washed with 250 µl of the washing solution.
(10-6) Fluorescence Detection
To the wells prepared in step (10-5) was added 1xSSPE at 100 µl per well. Measurement was performed with a fluorescence plate reader, Fluoroscan II (Dainippon Pharmaceutical) at the excitation wavelength of 584 nm and the observation wavelength of 612 nm. The results are shown below (the mean±SD values).

| target nucleic acid A | one base excessive sequence | one base deficient sequence | no addition of nucleic acid |
|---|---|---|---|
| 0.133±0.005 | 0.115±0.006 | 0.094±0.010 | 0.080±0.013 |

Target nucleic acid A clearly gave a larger value relative to the case of no addition of nucleic acid. The one base excessive sequence and the one base deficient sequence, which were both variant nucleic acids, showed values slightly larger than that of the case of no addition of nucleic acid, but significantly smaller than that of target nucleic acid A. These experimental results demonstrated that the presence or the absence of a nucleic acid could be detected.

### (11) Detection of Target Nucleic Acid Using Probe Labeled with Digoxigenin (Enzyme-Labeled Antibody Technique (Fluorescence))

(11-1) Immobilization of Probes
Probe 1, probe 2G, and target nucleic acid (each 100 nM) were mixed in 1xSSPE and heated at 95 ° C for 5 min to cause denaturation. Subsequently, they were maintained at 55 ° C for 10 min to effect hybridization. This hybrid was added to a 96-well microtiter plate in black coated with streptavidin at 94 µl per well. With agitating reaction was allowed to proceed at 37 ° C for 30 min. After the respective wells were three times washed with 250 µl of a washing solution attached to a NORTHERN ELISA, 10 mM aqueous sodium hydroxide solution containing 0.1% sodium dodecyl sulfate was added to the well at 100 µl per well and reaction was allowed at 37 ° C for 10 min. This caused target nucleic acid A to be dissociated from the probes.
(11-2) Hybridization of Target Nucleic Acid A and Variant Nucleic Acids Thereof
To the respective wells that immobilized the probes in step (11-1) were added target nucleic acid A, the one base excessive sequence and the one base deficient sequence--variant nucleic acids thereof--(each 200 nM) at 100 µl per well. After allowing to react in 1xSSPE at 37 ° C for 30 min, the respective wells were three times washed with 250 µl of the washing solution.
(11-3) Ligase Reaction on Solid Phase
To the wells prepared in step (11-2) was added T4 DNA ligase diluted with a reaction buffer as attached (which contained 30 IU ligase per well) and they were allowed to react at 37 ° C for 30 min. After the respective wells were three times washed with 250 µl of the washing solution, 10 mM aqueous sodium hydroxide solution containing 0.1% sodium dodecyl sulfate was added to the wells at 100 µl per well. The reaction was allowed to proceed at 37 ° C for 10 min, causing target nucleic acid A and its variant nucleic acids to be dissociated from the probes.
(11-4) Cleavage of the Disulfide Bond by DTT
To the wells prepared in step (11-3) was added 1xSSPE containing 500 mM DTT and 0.1% sodium dodecyl sulfate at 100 µl per well. After allowing to react at room temperature for 30 min, the wells were three times washed with 250 µl of the washing solution.
(11-5) Detection of Digoxigenin
To the wells prepared in step (11-4) was added alkaline phosphotase-labeled anti-digoxigenin antibody (Boehringer Manheim: 100 µl containing 7.5 mU antibody per well). After allowing to react at 37 ° C for 30 min, the respective wells were three times washed with 250 µl of the washing solution. Attophos (Boehringer Manheim), which acted as an alkaline phosphotase substrate to give a fluorescent product, was added to the wells at 100 µl per well and reaction was allowed to proceed at 37 ° C for 30 min. Then measurement was performed with a fluorescence plate reader, Fluoroscan II at the excitation wavelength of 485 nm and the observation wavelength of 590 nm. The results are shown below (the mean of four wells±SD values).

| target nucleic acid A | one base excessive sequence | one base deficient sequence | no addition of nucleic acid |
|---|---|---|---|
| 230.4±15.2 | 159.3±6.2 | 132.0±9.3 | 63.5±2.8 |

Target nucleic acid A clearly gave a larger value relative to the case of no addition of nucleic acid. The one base excessive sequence and the one base deficient sequence, which were both variant nucleic acids, showed values slightly larger than that in the case of no addition of nucleic acid, but significantly smaller than that of target nucleic acid A. These experimental results demonstrated that the presence or the absence of a nucleic acid could be detected.

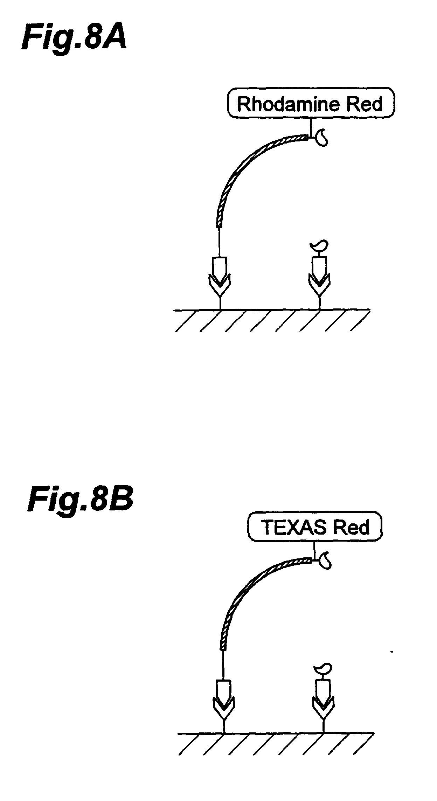

### (12) Detection of Target Nucleic Acid Using Probe Labeled with Rhodamine Red (Fig. 8A)

(12-1) Probe 2K comprising an oligonucleotide of 22 bases the 5'-terminus of which was biotinylated and the oligonucleotide chain of which was subjected to disulfide bond and Rhodamine Red (denoted [RR]) modification where two thymidine bases were introduced between the biotin and the disulfide bond, 5'-(biotin)-TT-[SS]-[RR]-GGTGGCGGCCGCTCTAGAAC-3', was automatically synthesized by the conventional phosphoramidite solid phase synthetic method.
(12-2) Immobilization of Probes
Probe 1, probe 2K, and target nucleic acid A (each 750 nM) were mixed in 1xSSPE and heated at 95 ° C for 5 min to cause denaturation. Subsequently, they were maintained at 55 ° C for 10 min to effect hybridization. This hybrid was added to a 96-well microtiter plate in black coated with streptavidin at 94 µl per well. With agitating reaction was allowed to proceed at 37 ° C for 30 min. After the respective wells were three times washed with 250 µl of a washing solution attached to a NORTHERN ELISA, 20 mM aqueous sodium hydroxide solution was added to the wells at 100 µl per well. The reaction was allowed to proceed at 37 ° C for 7 min, causing target nucleic acid A to be dissociated from the probes.
(12-3) Hybridization of Target Nucleic Acid A and Variant Nucleic Acids Thereof
To the respective wells that immobilized the probes in step (12-2) were added target nucleic acid A, the one base excessive sequence and the one base deficient sequence--variant nucleic acids thereof--(each 1.5 µM) at 100 µl per well. After allowing to react in 1xSSPE at 37 ° C for 30 min, the wells were three times washed with 250 µl of the washing solution.
(12-4) Ligase Reaction on Solid Phase
To the wells prepared in step (12-3) was added Ampligase (available from Epicentre Technologies Inc.: a heat-resistant ligase; 100 µl contained 20 U ligase per well) diluted with a reaction buffer as attached and they were allowed to react at 37 ° C for 30 min. After the respective wells were four times washed with 250 µl of the washing solution, 20 mM aqueous sodium hydroxide solution was added to the wells at 100 µl per well. The reaction was allowed to proceed at 37 ° C for 7 min, causing target nucleic acid A and its variant nucleic acids to be dissociated from the probes.
(12-5) cleavage of the Disulfide Bond by DTT
To the wells prepared in step (12-4) was added 1xSSPE containing 400 mM DTT and 0.1% sodium dodecyl sulfate at 100 µl per well. After allowing to react at 37 ° C for 30 min, the wells were three times washed with 250 µl of the washing solution.
(12-6) Fluorescence Detection
To the wells prepared in step (12-5) was added 1xSSPE at 100 µl per well. Measurement was performed with a fluorescence plate reader, Fluoroscan II at the excitation wavelength of 584 nm and the observation wavelength of 612 nm. The results are shown below (the mean when n=4±SD values).

| target nucleic acid A | one base excessive sequence | one base deficient sequence | no addition of nucleic acid |
|---|---|---|---|
| 0.385±0.020 | 0.199±0.008 | 0.235±0.005 | 0.229±0.023 |

Target nucleic acid A gave a significant larger value relative to the case of no addition of nucleic acid. The one base excessive sequence and the one base deficient sequence, which were both variant nucleic acids, showed values almost equal to that in the case of no addition of nucleic acid, but significantly smaller than that of target nucleic acid A. These experimental results demonstrated that the presence or the absence of a nucleic acid could be detected.

### (13) Detection of Target Nucleic Acid Using Probe Labeled with TEXAS Red (Fig. 8B)

(13-1) Probe 2L comprising an oligonucleotide of 22 bases the 5'-terminus of which was biotinylated and the oligonucleotide chain of which was subjected to disulfide bond and TEXAS Red (denoted [TR]), a fluorescent dye, modification where two thymidine bases were introduced between the biotin and the disulfide bond, 5'-(biotin)-TT-[SS]-[TR]-GGTGGCGGCCGCTCTAGAAC-3', was automatically synthesized by the conventional phosphoramidite solid phase synthetic method.
(13-2) Immobilization of Probes
Probe 1, probe 2L, and target nucleic acid A (each 1 µM) were mixed in 1xSSPE and heated at 95 ° C for 5 min to cause denaturation. Subsequently, they were maintained at 55 ° C for 10 min to effect hybridization. This hybrid was added to a 96-well microtiter plate in black coated with streptavidin at 94 µl per well. With agitating reaction was allowed to proceed at 37 ° C for 30 min. After the respective wells were three times washed with 250 µl of a washing solution attached to a NORTHERN ELISA, 20 mM aqueous sodium hydroxide solution was added to the wells at 100 µl per well. The reaction was allowed to proceed at 37 ° C for 7 min, causing target nucleic acid A to be dissociated from the probes.
(13-3) Hybridization of Target Nucleic Acid A and Variant Nucleic Acids Thereof
To the respective wells that had immobilized the probes in step (13-3) were added target nucleic acid A, the one base excessive sequence and the one base deficient sequence--variant nucleic acids thereof--(each 2 µM) at 100 µl per well. After allowing to react in 1xSSPE at 37 ° C for 30 min, the wells were four times washed with 250 µl of the washing solution.
(13-4) Ligase Reaction on Solid Phase
To the wells prepared in step (13-3) was added Ampligase (100 µl contained 20 U ligase per well) diluted with a reaction buffer as attached and they were allowed to react at 37 ° C for 30 min. After the respective wells were four times washed with 250 µl of the washing solution, 20 mM aqueous sodium hydroxide solution was added to the wells at 100 µl per well. The reaction was allowed to proceed at 37 ° C for 7 min, causing target nucleic acid A and its variant nucleic acids to be dissociated from the probes.
(13-5) Cleavage of the Disulfide Bond by DTT
To the wells prepared in step (13-4) was added 1xSSPE containing 500 mM DTT and 0.1% sodium dodecyl sulfate at 100 µl per well. After allowing to react at 37 ° C for 50 min, the wells were three times washed with 250 µl of the washing solution.
(13-6) Fluorescence Detection
To the wells prepared in step (13-5) was added 1xSSPE at 100 µl per well. Measurement was performed with a fluorescence plate reader, Fluoroscan II at the excitation wavelength of 584 nm and the observation wavelength of 612 nm. The results are shown below (the mean when n=4±SD values).

| target nucleic acid A | one base excessive sequence | one base deficient sequence | no addition of nucleic acid |
|---|---|---|---|
| 0.128±0.010 | 0.054±0.007 | 0.062±0.004 | 0.058±0.011 |

Target nucleic acid A gave a significant larger value relative to the case of no addition of nucleic acid. The one base excessive sequence and the one base deficient sequence, which were both variant nucleic acids, showed values almost equal to that in the case of no addition of nucleic acid, but significantly smaller than that of target nucleic acid A. These experimental results demonstrated that the presence or the absence of a nucleic acid could be detected.

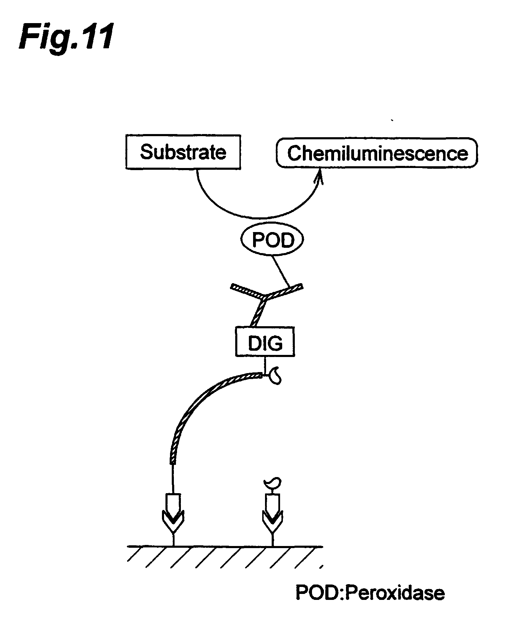

### (14) Detection of Target Nucleic Acid Using Probe Labeled with Digoxigenin (Enzyme-Labeled Antibody Technique (Chemluminescence) Fig. 11)

(14-1) Immobilization of Probes
Probe 1, probe 2G, and target nucleic acid A (each 100 pM) were mixed in 1xSSPE and heated at 95 ° C for 5 min to cause denaturation. Subsequently, they were maintained at 55 ° C for 10 min to effect hybridization. This hybrid was added to a 96-well microtiter plate in black coated with streptavidin at 94 µl per well. With agitating reaction was allowed to proceed at 37 ° C for 10 min. After the respective wells were three times washed with 250 µl of a washing solution attached to a NORTHERN ELISA, 20 mM aqueous sodium hydroxide solution was added to the wells at 100 µl per well. The reaction was allowed to proceed at 37 ° C for 5 min, causing target nucleic acid A to be dissociated from the probes.
(14-2) Hybridization of Target Nucleic Acid A and Variant Nucleic Acids Thereof
To the respective wells that immobilized the probes in step (14-1) were added target nucleic acid A, the one base excessive sequence and the one base deficient sequence--variant nucleic acids thereof--(each 1 nM) at 100 µl per well. After allowing to react in 1xSSPE at 37 ° C for 60 min, the wells were four times washed with 250 µl of the washing solution.
(14-3) Ligase Reaction on Solid Phase
To the wells prepared in step (14-2) was added Ampligase (100 µl contained 10 U ligase per well) and they were allowed to react at 37 ° C for 15 min. Subsequently, the wells were four times washed with 250 µl of the washing solution.
(14-4) Cleavage of the Disulfide Bond by DTT
To the wells prepared in step (14-3) was added 0.1 M Tris-HCl buffer (pH 8.0) containing 400 mM DTT and 0.1% sodium dodecyl sulfate at 100 µl per well, and reaction was allowed to proceed at 37 ° C for 30 min. After the respective wells were four times washed with 250 µl of the washing solution, 20 mM aqueous sodium hydroxide solution was added to the wells at 100 µl per well. The reaction was allowed to proceed at 37 ° C for 5 min, causing target nucleic acid A and its variant nucleic acids to be dissociated from the probes.
(14-5) Detection of Digoxigenin
To the wells prepared in step (14-4) was added peroxidase-labeled anti-digoxigenin antibody dissolved in a phosphate buffer solution containing 1% bovine serum albumin (100 µl containing 5 mU antibody per well). After allowing to react at 37 ° C for 30 min, the respective wells were four times washed with 250 µ 1 of the washing solution. Substrate Reagent A (which contained luminol and 4-iodophenol: 99 µl per well) and Starting Reagent B (which contained hydrogen peroxide: 1 µl per well) as attached to a BM Chemiluminescence ELISA Reagent (Boehringer Manheim) were added to the wells and reaction was allowed to proceed at room temperature for 5 min. Measurement was then performed with a luminescence plate reader, MRL 100 (Corona Industry). The results are shown below (the mean when n=4±SD values).

| target nucleic acid A | one base excessive sequence | one base deficient sequence | no addition of nucleic acid |
|---|---|---|---|
| 20556±442 | 1487±84 | 1757±114 | 1394±113 |

Target nucleic acid A gave a significant larger value relative to the case of no addition of nucleic acid. The one base excessive sequence and the one base deficient sequence, which were both variant nucleic acids, showed values larger than that in the case of no addition of nucleic acid, but smaller than that of target nucleic acid A. These experimental results demonstrated that the presence or the absence of a nucleic acid could be detected.

### (15) Detection of Target Nucleic Acid with Probe Having Deoxyuridine Introduced as the Cleavage Part

(15-1) Probe 2M comprising an oligonucleotide of 25 bases the 5'-terminus of which was biotinylated and the oligonucleotide chain of which was subjected to deoxyuridine (denoted "dU") and digoxigenin modification, 5'-(biotin)-dUdUdUdUdU-[DIG]-GGTGGCGGCCGCTCTAGAAC-3', was automatically synthesized by the conventional phosphoramidite solid phase synthetic method.
(15-2) A target nucleic acid A complementary sequence comprising an oligonucleotide of 40 bases, 5'-GGTGGCGGCCGCTCTAGAACTAGTGGATCCCCCGGGCTGC-3', was automatically synthesized by the conventional phosphoramidite solid phase synthetic method.
(15-3) Immobilization of Probes
Probe 1, probe 2M, and target nucleic acid A (each 500 pM) were mixed in 1xSSPE and heated at 95 ° C for 5 min to cause denaturation. Subsequently, they were maintained at 55 ° C for 10 min to effect hybridization. This hybrid was added to a 96-well microtiter plate coated with streptavidin as attached to a NORTHERN ELISA at 94 µl per well. With agitating reaction was allowed to proceed at 37 ° C for 10 min. After the respective wells were three times washed with 250 µl of a washing solution attached to the NORTHERN ELISA, 20 mM aqueous sodium hydroxide solution was added to the wells at 100 µl per well. The reaction was allowed to proceed at 37 ° C for 5 min, causing target nucleic acid A to be dissociated from the probes.
(15-4) Hybridization of Target Nucleic Acid A and Target Nucleic Acid A Complementary Sequence
To the respective wells that had immobilized the probes in step (15-3) were added target nucleic acid A and the target nucleic acid A complementary sequence comprising a sequence complementary thereto (each 1 nM) at 100 µl per well. After allowing to react in 1xSSPE at 37 ° C for 60 min, the wells were four times washed with 250 µl of the washing solution.
(15-5) Ligase Reaction on Solid Phase
To the wells prepared in step (15-4) was added Ampligase (100 µl contained 10 U ligase per well) diluted with the reaction buffer as attached and they were allowed to react at 37 ° C for 15 min. After the respective wells were four times washed with 250 µl of the washing solution, 20 mM aqueous sodium hydroxide solution was added to the wells at 100 µl per well. The reaction was allowed to proceed at 37 ° C for 5 min, causing target nucleic Acid A and its complementary sequence to be dissociated from the probes.
(15-6) Cleavage of the Deoxyuridine Chain by Uracil DNA Glycosidase
To the wells prepared in step (15-5) was added uracil DNA glycosidase diluted with a reaction buffer as attached (Amasham: 100 µl contained 5 U enzyme per well) and they were allowed to react at room temperature for 30 min. After the respective wells were four times washed with 250 µl of the washing solution, 1.0 M Tris-HCl buffer (pH 10.0) was added to the wells at 100 µl per well and reaction was allowed to proceed at 70 ° C for 10 min. After the wells were four times washed with 250 µl of the washing solution, 20 mM aqueous sodium hydroxide solution was added to the wells at 100 µl per well. The reaction was allowed to proceed at 37 ° C for 5 min, causing target nucleic acid A and the target nucleic acid A complementary sequence to be dissociated from the probes.
(15-7) Detection of Digoxigenin
To the wells prepared in step (15-6) was added Anti-DIG-POD (100 µl contained 5 mU antibody per well) and reaction was allowed to proceed at 37 ° C for 30 min with shaking. After the respective wells were four times washed with 250 µl of the washing solution, the wells were allowed to react with a TMB Substrate Solution attached to the NORTHERN ELISA (100 µl per well added) at room temperature for 10 min. The reaction was quenched with a Stop Reagent attached to the NORTHERN ELISA (100 µl per well added). Absorbance at 460 nm was measured with a microplate reader, SPECTRA MAX 250; the results are shown in the table below (the mean when n=4±SD values).

| target nucleic acid A | target nucleic acid A complementary sequence | no addition of nucleic acid |
|---|---|---|
| 0.383±0.018 | 0.099±0.011 | 0.119±0.004 |

The target nucleic acid A complementary sequence, a variant nucleic acid, showed a value similar to that of the wells where no nucleic acid was added, and only target nucleic acid A showed a significant large value. These experimental results demonstrated that even where deoxyuridine was selected to be the cleavage part, cleavage (scission) took place and that even when a probe to which deoxyuridine had been introduced as a cleavage part was used, the presence or the absence of the target nucleic acid could be detected.

### (16) Detection of Long Chain Target Nucleic Acid (100 Bases)

(16-1) Target nucleic acid B comprising an oligonucleotide of 100 bases containing target nucleic acid at a center thereof, 5'-CGGTATCGATAAGCTTGATATCGAATTCCTGCAGCCCGGGGGATCCACTAGTTCT AGAGCGGCCGCCACCGCGGTGGAGCTCCAATTCGCCCTATAGTGA-3', was automatically synthesized by the conventional phosphoramidite solid phase synthetic method.
(16-2) Immobilization of Probes
Probe 1, probe 2G, and target nucleic acid A (each 1 nM) were mixed in 1xSSPE and heated at 95 ° C for 5 min to cause denaturation. Subsequently, they were maintained at 55 ° C for 10 min to effect hybridization. This hybrid was added to a 96-well microtiter plate in black coated with streptavidin as attached to a NORTHERN ELISA at 94 µl per well. With shaking reaction was allowed to proceed at 37 ° C for 10 min. After the respective wells were twice washed with 250 µl of a washing solution attached to the NORTHERN ELISA, 20 mM aqueous sodium hydroxide solution was added to the wells at 100 µl per well. The reaction was allowed to proceed at 37 ° C for 5 min, causing target nucleic acid A to be dissociated from the probes.
(16-3) Hybridization of Target Nucleic Acid B and Target Nucleic Acid A
To the wells that immobilized the probes in step (16-2) were added target nucleic acid A and target nucleic acid B (each 1 nM) at 100 µl per well. After allowing to react in 1xSSPE at 60 ° C for 60 min, the respective wells were four times washed with 250 µl of the washing solution.
(16-4) Ligase Reaction on Solid Phase
To the wells prepared in step (16-3) was added Ampligase (100 µl contained 10 U ligase per well) diluted with the reaction buffer as attached. After allowing to react at 60 ° C for 10 min, the respective wells were four times washed with 250 µl of the washing solution.
(16-5) Cleavage of the Disulfide Bond by DTT
To the wells prepared in step (16-4) was added 0.1 M Tris-HCL buffer (pH 8.0) containing 400 mM DTT and 0.1% sodium dodecyl sulfate at 100 µl per well and they were allowed to react at 60 ° C for 30 min. After the respective wells were five times washed with 250 µ l of the washing solution, 20 mM aqueous sodium hydroxide solution was added to the wells at 100 µl per well. The reaction was allowed to proceed at 60 ° C for 5 min, causing target nucleic acid B and target nucleic acid A to be dissociated from the probes.
(16-6) Detection of Digoxigenin
To the wells prepared in step (16-5) was added Anti-DIG-POD (100 µl containing 5 mU antibody per well ) as attached to the NORTHERN ELISA and reaction was allowed to proceed at 37 ° C for 30 min with shaking. After the rspective wells were four times washed with 250 µl of the washing solution, the well was allowed to react with a TMB Substrate Solution attached to the NORTHERN ELISA (100 µl per well added) at room temperature for 10 min. The reaction was quenched with a Stop Reagent attached to the NORTHERN ELISA (100 µl per well added). Absorbance at 460 nm was measured with a microplate reader, SPECTRA MAX 250; the results are shown in the table below (the mean when n=4±SD values).

| target nucleic acid B (100 bases) | target nucleic acid A (40 bases) | no addition of nucleic acid |
|---|---|---|
| 0.171±0.009 | 0.449±0.017 | 0.011±0.001 |

Target nucleic acid B (100 bases) gave a significant larger value relative to the case of no addition of nucleic acid. Consequently, it was demonstrated that the detection could be done even if the target nucleic acid contained a sequence other than the sequence which the probes would recognize.

### (17) Detection of Double-Stranded Target Nucleic Acid

(17-1) Immobilization of Probes
Probe 1, probe 2G, and target nucleic acid A (each 1 nM) were mixed in 1xSSPE and heated at 95 ° C for 5 min to cause denaturation. Subsequently, they were maintained at 55 ° C for 10 min to effect hybridization. This hybrid was added to a 96-well microtiter plate in black coated with streptavidin as attached to a NORTHERN ELISA at 94 µl per well. With shaking reaction was allowed to proceed at 37 ° C for 10 min. After the respective wells were four times washed with 250 µl of a washing solution attached to the NORTHERN ELISA, 20 mM aqueous sodium hydroxide solution was added to the wells at 100 µl per well. The reaction was allowed to proceed at 37 ° C for 5 min, causing target nucleic acid A to be dissociated from the probes.
(17-2) Hybridization of Target Nucleic Acid A and A Variant Nucleic Acid Thereof
To the respective wells that immobilized the probes in step (17-1) was added 2xSSPE at 90 µl per well. Subsequently, 0.01xSSPE containing each (1 nM) of a double-stranded nucleic acid comprising target nucleic acid A and the target nucleic acid A-complementary sequence, target nucleic acid A (positive control), and the target nucleic acid A complementary sequence (negative control) was added to the wells at 10 µl per well (with final concentrations of 100 pM). After allowing the reaction to proceed at 37 ° C for 60 min, the wells was four times washed with 250 µl of the washing solution.
(17-3) Ligase Reaction on Solid Phase
To the wells prepared in step (17-2) was added Ampligase (100 µl contained 10 U ligase per well) diluted with a reaction buffer as attached. After allowing to react at 37 ° C for 15 min, the respective wells were four times with 250 µl of the washing solution.
(17-4) Cleavage of the Disulfide Bond by DTT
To the wells prepared in step (17-3) was added 0.1 M Tris-HCl buffer (pH 8.0) containing 400 mM DTT and 0.1% sodium dodecyl sulfate at 100 µl per well and they were allowed to react at 37 ° C for 30 min. After the respective wells were four times washed with 250 µ l of the washing solution, 20 mM aqueous sodium hydroxide solution was added to the wells at 100 µl per well. The reaction was allowed to proceed at 37 ° C for 5 min, causing the double-stranded nucleic acid and the control nucleic acids to be dissociated from the probes.
(17-5) Detection of Digoxigenin
To the wells prepared in step (17-4) was added Anti-DIG-POD (100 µl containing 5 mU antibody per well ) and reaction was allowed to proceed at 37 ° C for 30 min with shaking. After the respective wells were four times washed with 250 µl of the washing solution, the wells were allowed to react with a TMB Substrate Solution attached to the NORTHERN ELISA (100 µl per well added) at room temperature for 10 min. The reaction was quenched with a Stop Reagent attached to the NORTHERN ELISA (100 µl per well added). Absorbance at 460 nm was measured with a microplate reader, SPECTRA MAX 250; the results are shown in the table below (the mean when n=4±SD values).

| double-stranded nucleic acid | target nucleic acid A | target nucleic acid-complementary sequence | no addition of nucleic acid |
|---|---|---|---|
| 0.084±0.006 | 0.095±0.006 | 0.044±0.004 | 0.039±0.002 |

The double-stranded nucleic acid gave a significantly larger value relative to the cases of no addition of nucleic acid and the target nucleic acid A complementary sequence, which was then equal to that of target nucleic acid A. These experimental results demonstrated that even where a double-stranded sample was used, the target nucleic acid could be detected.

### INDUSTRIAL APPLICABILITY

Probes 1 and 2, and the solid phases of this invention have structures as explained above. Accordingly, when a target nucleic acid is hybridized above the solid phase of the invention, the probes on the solid phase occupy spatial positions beneficial to the formation of a hybrid and thus forms the hybrid efficiently. Therefore, probe 1 and probe 2 are efficiently ligated by ligase reaction. Furthermore, after the target nucleic acid is removed from the hybrid, only probe 2 ligated as described above will be able to exist on the solid phase through the cleavage reaction of a cleavage part.

Accordingly, after the free probe 2 has been removed by washing, it will become possible to detect the presence of probe 2 bound on the solid phase as described above, with high sensitivity and high recognition. This will enable detection of the presence of a target nucleic acid with higher sensitivity and higher recognition as compared to methods in the prior art.

## Claims

1. A pair of probes for the detection of a target nucleic acid comprising:
(1) probe 1 having base sequence B1 complementary to A1 between two specific sequential base sequences A1 and A2 of the target nucleic acid, wherein (a) the 5'-terminus of base sequence B1 is phosphorylated and (b) a first solid phase immobilizing part is bound to the 3'-terminus of base sequence B1; and
(2) probe 2 having base sequence B2 complementary to A2 between base sequences A1 and A2, wherein (a) the 5'-terminus of base sequence B2 is bound to one end of a cleavage part and (b) a second solid phase immobilizing part is bound to the other part of the cleavage part.

2. A pair of probes for the detection of a target nucleic acid comprising:
(1) probe 1 having base sequence B1 complementary to A1 between two specific sequential base sequences A1 and A2 of the target nucleic acid, wherein (a) the 5'-terminus of base sequence B1 is phosphorylated and (b) the 3'-terminus of base sequence B1 is bound to one end of a cleavage part and (c) a first solid phase immobilizing part is bound to the other end of the cleavage part; and
(2) probe 2 having base sequence B2 complementary to A2 between base sequences A1 and A2, wherein a second solid phase immobilizing part is bound to the 5'-terminus of base sequence B2.

3. The probes for the detection of a target nucleic according to claim 1, wherein probe 2 is further provided with a labeling part between the 5'-terminus of base sequence B2 and the cleavage part.

4. The probes for the detection of a target nucleic acid according to claim 2, wherein probe 1 is further provided with a labeling part between the 3'-terminus of base sequence B1 and the cleavage part.

5. The probes for the detection of a target nucleic acid according to any of claims 1 - 4, wherein the cleavage part has a disulfide bond.

6. A solid phase for the detection of a target nucleic acid comprising:
(1) probe 1 having base sequence B1 complementary to A1 between two specific sequential base sequences A1 and A2 of the target nucleic acid, wherein (a) the 5'-terminus of base sequence B1 is phosphorylated and (b) a first solid phase immobilizing part is bound to the 3'-terminus of base sequence B1; and
(2) probe 2 having base sequence B2 complementary to A2 between base sequences A1 and A2, wherein (a) the 5'-terminus of base sequence B2 is bound to one end of a cleavage part and (b) a second solid phase immobilizing part is bound to the other end of the cleavage part,
wherein probe 1 is immobilized to a surface by the first solid phase immobilizing part and probe 2 is immobilized to the surface by the second solid phase immobilizing part.

7. A solid phase for the detection of a target nucleic acid comprising:
(1) probe 1 having base sequence B1 complementary to A1 between two specific sequential base sequences A1 and A2 of the target nucleic acid, wherein (a) the 5'-terminus of base sequence B1 is phosphorylated, (b) the 3'-terminus of base sequence B1 is bound to one end of a cleavage part, and (c) a first solid phase immobilizing part is bound to the other end of the cleavage part; and
(2) probe 2 having base sequence B2 complementary to A2 between base sequences A1 and A2, wherein a second solid phase immobilizing part is bound to the 5'-terminus of base sequence B2,
wherein probe 1 is immobilized to a surface by the first solid phase immobilizing part and probe 2 is immobilized to the surface by the second solid phase immobilizing part.

8. The solid phase for the detection of a target nucleic acid according to claim 6, wherein probe 2 is further provided with a labeling part between the 5'-terminus of base sequence B2 and the cleavage part.

9. The solid phase for the detection of a target nucleic acid according to claim 7, wherein probe 1 is further provided with a labeling part between the 3'-terminus of base sequence B1 and the cleavage part.

10. The solid phase for the detection of a target nucleic acid according to any of claims 6 - 9, wherein the cleavage part has a disulfide bond.

11. A method of detecting a target nucleic acid comprising:
(A) a first step of immobilizing probe 1 to a surface of a solid phase through a first solid phase immobilizing part and immobilizing probe 2 to the surface of the solid phase through a second solid phase immobilizing part;
wherein (1) probe 1 has base sequence B1 complementary to A1 between two specific sequential base sequences A1 and A2 of the target nucleic acid, further wherein (a) the 5'-terminus of base sequence B1 is phosphorylated and (b) the first solid phase immobilizing part is bound to the 3'-terminus of base sequence B1, and wherein (2) probe 2 has base sequence B2 complementary to A2 between base sequences A1 and A2, further wherein (a) the 5'-terminus of base sequence B2 is bound to one end of a cleavage part and (b) the second solid phase immobilizing part is bound to the other end of the cleavage part,
(B) a second step of hybridizing probe 1, probe 2, and the target nucleic acid to form a hybrid;
(C) a third step of ligating probe 1 and probe 2 in the hybrid by a ligase reaction;
(D) a fourth step of cleaving the cleavage part of probe 2 by a cleavage reaction; and
(E) a fifth step of detecting probe 1 which has been formed in the third step and which has bound base sequence B2 and the cleavage part of probe 2.

12. A method of detecting a target nucleic acid comprising:
(A) a first step of immobilizing probe 1 to a surface of a solid phase through a first solid phase immobilizing part and immobilizing probe 2 to the surface of the solid phase through a second solid phase immobilizing part;
wherein (1) probe 1 has base sequence B1 complementary to A1 between two specific sequential base sequences A1 and A2 of the target nucleic acid, further wherein (a) the 5'-terminus of base sequence B1 is phosphorylated, (b) a cleavage part is bound to the 3'-terminus of base sequence B1, and (c) the first solid phase immobilizing part is bound to the other end of the cleavage part, and wherein (2) probe 2 has base sequence B2 complementary to A2 between base sequences A1 and A2, further wherein the second solid phase immobilizing part is bound to the 5'-terminus of base sequence B2,
(B) a second step of hybridizing probe 1, probe 2, and the target nucleic acid to form a hybrid;
(C) a third step of ligating probe 1 and probe 2 in the hybrid by a ligase reaction;
(D) a fourth step of cleaving the cleavage part of probe 1 by a cleavage reaction; and
(E) a fifth step of detecting probe 2 which has been formed in the third step and has bound base sequence B1 and the cleavage part of probe 1.

13. The method of detecting a target nucleic acid according to claim 11, wherein probe 2 is further provided with a labeling part between the 5'-terminus of base sequence B2 and the cleavage part.

14. The method of detecting a target nucleic acid according to claim 12, wherein probe 1 is further provided with a labeling part between the 3'-terminus of base sequence B1 and the cleavage part.

15. The method of detecting a target nucleic acid according to any of claims 11 - 14, wherein the cleavage part has a disulfide bond and the disulfide bond is cleaved with a reducing agent in the fourth step.

16. The method of detecting a target nucleic acid according to any of claims 11 - 15, wherein the mass of the probe bound to the solid phase is detected by surface plasmon resonance spectroscopy in the fifth step.

17. A method of preparing a solid phase for the detection of a target nucleic acid, said method comprising:
(A) a first step of hybridizing (1) two specific sequential base sequences A1 and A2 of the target nucleic acid, (2) probe 1 having base sequence B1 complementary to A1 between the two specific sequential base sequences A1 and A2, wherein (a) the 5'-terminus of base sequence B1 is phosphorylated and (b) a first solid phase immobilizing part is bound to the 3'-terminus of base sequence B1, and (3) probe 2 having base sequence B2 complementary to A2 between base sequences A1 and A2, wherein (a) the 5'-terminus of base sequence B2 is bound to one end of a cleavage part and (b) a second solid phase immobilizing part is bound to the other end of the cleavage part, to form a hybrid;
(B) a second step of immobilizing the hybrid to a surface of a solid phase through the first and second solid phase immobilizing parts; and
(C) a third step of removing the target nucleic acid from the hybrid.

18. A method of preparing a solid phase for the detection of a target nucleic acid, said method comprising:
(A) a first step of hybridizing (1) two specific sequential base sequences A1 and A2 of the target nucleic acid, (2) probe 1 having base sequence B1 complementary to A1 between the two specific sequential base sequences A1 and A2, wherein (a) the 5'-terminus of base sequence B1 is phosphorylated, (b) the 3'-terminus of base sequence B1 is bound to one end of a cleavage part, and (c) a first solid phase immobilizing part is bound to the other end of the cleavage part, and (3) probe 2 having base sequence B2 complementary to A2 between base sequences A1 and A2, wherein a second solid phase immobilizing part is bound to the 5'-terminus of base sequence B2, to form a hybrid;
(B) a second step of immobilizing the hybrid to a surface of a solid phase through the first and second solid phase immobilizing parts; and
(C) a third step of removing the target nucleic acid from the hybrid.


SEQUENCE LISTING
<110>Laboratory of Molecular Biophotonics

<120>Probes for detecting a target nucleic acid, methods for detecting a target
nucleic acid, and a solid material for detecting a target nucleic acid and method
for preparing the same

<130>BBP98-03PCT

<160>20

<210>1
<211>20
<212>DNA
<213>Artificial Sequence

<220>
<223>a probe, 5'-terminal:Biotin

<400>1
GGTGGCGGCC GCTCTAGAAC 20

<210>2
<211>23
<212>DNA
<213>Artificial Sequence

<220>
<223>a probe, 5'-terminal:Biotin, N:Disulfide bond

<400>2
TTNGGTGGCG GCCGCTCTAG AAC 23

<210>3
<211>26
<212>DNA
<213>Artificial Sequence

<220>
<223>a probe, 5'-terminal:Biotin, N:Disulfide bond

<400>3
TTTTTNGGTG GCGGCCGCTC TAGAAC 26

<210>4

<211>31
<212>DNA
<213>Artificial Sequence

<220>
<223>a probe, 5'-terminal:Biotin, N:Disulfide bond

<400>4
TTTTTTTTTT NGGTGGCGGC CGCTCTAGAA C 31

<210>5
<211>20
<212>DNA
<213>Artificial Sequence

<220>
<223>a probe, 5'-terminal:Phosphorylated, 3'-terminal:Biotin

<400>5
TAGTGGATCC CCCGGGCTGC 20

<210>6
<211>40
<212>DNA
<213>Artificial Sequence

<220>
<223>a target

<400>6
GCAGCCCGGG GGATCCACTA GTTCTAGAGC GGCCGCCACC 40

<210>7
<211>22
<212>DNA
<213>Artificial Sequence

<220>
<223>a probe,5'-terminal:Biotin, N1:Disulfide bond, N2:BODIPY493/503

<400>7
NNGGTGGCGG CCGCTCTAGA AC 22

<210>8
<211>41

<212>DNA
<213>Artificial Sequence

<220>
<223>a target

<400>8
GCAGCCCGGG GGATCCACTA AGTTCTAGAG CGGCCGCCAC C 41

<210>9
<211>39
<212>DNA
<213>Artificial Sequence

<220>
<223>a target

<400>9
GCAGCCCGGG GGATCCACTG TTCTAGAGCG GCCGCCACC 39

<210>10
<211>23
<212>DNA
<213>Artificial Sequence

<220>
<223>a probe,5'-terminal:Biotin, N:Digoxigenin

<400>10
TTNGGTGGCG GCCGCTCTAG AAC 23

<210>11
<211>24
<212>DNA
<213>Artificial Sequence

<220>
<223>a probe,5'-terminal:Biotin, N1:Disulfide bond, N2:Digoxigenin

<400>11
TTNNGGTGGC GGCCGCTCTA GAAC 24

<210>12
<211>22
<212>DNA

<213>Artificial Sequence

<220>
<223>a probe,5'-terminal:Biotin, N1:Disulfide bond, N2:Digoxigenin

<400>12
NNGGTGGCGG CCGCTCTAGA AC 22

<210>13
<211>27
<212>DNA
<213>Artificial Sequence

<220>
<223>a probe,5'-terminal:Biotin, N1:Disulfide bond, N2:Digoxigenin

<400>13
TTTTTNNGGT GGCGGCCGCT CTAGAAC 27

<210>14
<211>32
<212>DNA
<213>Artificial Sequence

<220>
<223>a probe,5'-terminal:Biotin, N1:Disulfide bond, N2:Digoxigenin

<400>14
TTTTTTTTTT NNGGTGGCGG CCGCTCTAGA AC 32

<210>15
<211>24
<212>DNA
<213>Artificial Sequence

<220>
<223>a probe,5'-terminal:Biotin, N1:Disulfide bond, N2:Rhodamine Red

<400>15
TTNNGGTGGC GGCCGCTCTA GAAC 24

<210>16
<211>24
<212>DNA
<213>Artificial Sequence

<220>
<223>a probe,5'-terminal:Biotin, N1:Disulfide bond, N2:TEXAS Red

<400>16
TTNNGGTGGC GGCCGCTCTA GAAC 24

<210>17
<211>26
<212>DNA
<213>Artificial Sequence

<220>
<223>a probe,5'-terminal:Biotin, N1-5:Deoxyuridine, N6:Digoxigenin

<400>17
NNNNNNGGTG GCGGCCGCTC TAGAAC 26

<210>18
<211>40
<212>DNA
<213>Artificial Sequence

<220>
<223>a target

<400>18
GGTGGCGGCC GCTCTAGAAC TAGTGGATCC CCCGGGCTGC 40

<210>19
<211>100
<212>DNA
<213>Artificial Sequence

<220>
<223>a target

<400>19
CGGTATCGAT AAGCTTGATA TCGAATTCCT GCAGCCCGGG GGATCCACTA GTTCTAGAGC 60
GGCCGCCACC GCGGTGGAGC TCCAATTCGC CCTATAGTGA 100